# EUROPEAN PATENT APPLICATION

(11) **EP 2 292 742 A1**
(43) Date of publication of application: **09.03.2011**
(21) Application number: 09011140.2
(22) Date of filing: 31.08.2009
(51) Int. Cl.: C12N 9/10

(54) **New nucleic acid molecules and polypeptides involved in lipid metabolism**

(71) Applicant: Rheinische Friedrich-Wilhelms-Universität Bonn, 53113 Bonn (DE)
(72) Inventor: Hoch, Michael Karl, 53913 Swisttal (DE); Bauer, Reinhard, 53123 Bonn (DE); Schultze, Joachim, 50259 Pulheim-Simmersdorf (DE); Völzmann, André, 53913 Swisttal (DE)
(74) Representative: Kröncke, Rolf

(57) **Abstract**

The present invention relates in a first aspect to polypeptides useful for the prevention or treatment of various diseases. In particular, the present invention relates to a polypeptide containing a homeodomain for the prevention or treatment of lipid-based metabolic diseases, cancer, (neuro)degeneration, autoimmunity, sepsis, cystic fibrosis, diabetes, or for modulating the immune system in an individual or as an anti aging compound. In another aspect, the present invention relates to nucleic acids encoding said polypeptides, vectors containing said nucleic acid molecules as well as host cells comprising said vector. Further, the present invention concerns isolated antisense RNA or DNA molecules. In addition, the present invention provides methods for screening compounds for treating or preventing said diseases based on the ability to alter expression of ceramide synthases and its cleavage.

SEQUENCE LISTING

<110>  Rheinische Friedrich-Wilhelmx Universität Bonn

<120>  new nucleic acid molecules and polypeptides involved in lipid
       metabolism

<130>  4464-001

<160>  41

<170>  PatentIn version 3.3

<210>  1
<211>  6
<212>  PRT
<213>  artificial

<220>
<223>  peptide containing a homeodomain motif


<220>
<221>  MISC_FEATURE
<222>  (3)..(3)
<223>  Xaa is any amino acid residue

<220>
<221>  MISC_FEATURE
<222>  (5)..(5)
<223>  Xaa any amino acid having a basic side chain

<400>  1

Arg Arg Xaa Gln Xaa Pro
1               5


<210>  2
<211>  10
<212>  PRT
<213>  artificial

<220>
<223>  peptide containing a homeodomain motif


<220>
<221>  MISC_FEATURE
<222>  (2)..(2)
<223>  Xaa is any amino acid

<220>
<221>  MISC_FEATURE
<222>  (4)..(4)
<223>  Xaa is any amino acid

<220>
<221>  MISC_FEATURE
<222>  (7)..(7)
<223>  Xaa is any amino acid

<220>
<221>  MISC_FEATURE
<222>  (9)..(9)
<223>  Xaa is any amino acid

<400>  2

Trp Xaa Arg Xaa Arg Arg Xaa Gln Xaa Pro
1               5                   10


<210>  3
<211>  52
<212>  PRT
<213>  artificial

<220>
<223>  polypeptide containg the Lag domain of ceramide synthase

<400>  3

Arg Lys Asp Phe Trp Gln Met Phe Ile His His Met Val Thr Leu Leu
1               5                   10                  15


Leu Met Ser Leu Ser Trp Val Cys Asn Leu His Arg Val Gly Ser Leu
            20                  25                  30


Val Leu Val Val His Asp Cys Ala Asp Ile Phe Leu Glu Ala Ala Lys
        35                  40                  45


Leu Thr Lys Tyr
    50


<210>  4
<211>  42
<212>  PRT
<213>  artificial

<220>
<223>  peptide containing a homeodomain motif


<220>
<221>  misc_features
<222>  (3)..(32)
<223>  Xaa is any amino acid

<220>
<221>  misc_features
<222>  (34)..(34)
<223>  Xaa is any amino acid

<220>
<221>  misc_features
<222>  (36)..(36)
<223>  Xaa is any amino acid

<220>
<221>  misc_features
<222>  (39)..(39)
<223>  Xaa is any amino acid

<220>
<221>  misc_features
<222>  (41)..(41)
<223>  Xaa is any amino acid

<400>  4

Leu Glu Xaa Xaa Xaa Xaa Xaa Xaa Xaa Xaa Xaa Xaa Xaa Xaa Xaa Xaa

1                   5                   10                  15

Xaa Xaa Xaa Xaa Xaa Xaa Xaa Xaa Xaa Xaa Xaa Xaa Xaa Xaa Xaa Xaa
            20                  25                  30

Trp Xaa Arg Xaa Arg Arg Xaa Gln Xaa Pro
        35                  40

<210>  5
<211>  43
<212>  PRT
<213>  artificial

<220>
<223>  peptide containing homeodomain motif

<220>
<221>  misc-features
<222>  (3)..(33)
<223>  xaa is any amino acid

<220>
<221>  misc-features
<222>  (35)..(35)
<223>  xaa is any amino acid

<220>
<221>  misc-features
<222>  (37)..(37)
<223>  xaa is any amino acid

<220>
<221>  misc-features
<222>  (40)..(40)
<223>  xaa is any amino acid

<220>
<221>  misc-features
<222>  (42)..(42)
<223>  xaa is any amino acid

<400>  5

Leu Glu Xaa Xaa Xaa Xaa Xaa Xaa Xaa Xaa Xaa Xaa Xaa Xaa Xaa Xaa
1               5                   10                  15

Xaa Xaa Xaa Xaa Xaa Xaa Xaa Xaa Xaa Xaa Xaa Xaa Xaa Xaa Xaa Xaa
            20                  25                  30

Xaa Trp Xaa Arg Xaa Arg Arg Xaa Gln Xaa Pro
        35                  40

<210>  6
<211>  61
<212>  PRT
<213>  human

<400>  6

Asn Ile Lys Glu Lys Thr Arg Leu Arg Ala Pro Pro Asn Ala Thr Leu
1               5                   10                  15

Glu His Phe Tyr Leu Thr Ser Gly Lys Gln Pro Lys Gln Val Glu Val
            20                  25                  30

Glu Leu Leu Ser Arg Gln Ser Gly Leu Ser Gly Arg Gln Val Glu Arg
        35                  40                  45

Trp Phe Arg Arg Arg Arg Asn Gln Asp Arg Pro Ser Leu
    50                  55                  60

<210>  7
<211>  60
<212>  PRT
<213>  human

<400>  7

Gly Ile Lys Glu Thr Val Arg Lys Val Thr Pro Asn Thr Val Leu Glu
1               5                   10                  15

Asn Phe Phe Lys His Ser Thr Arg Gln Pro Leu Gln Thr Asp Ile Tyr
            20                  25                  30

Gly Leu Ala Lys Lys Cys Asn Leu Thr Glu Arg Gln Val Glu Arg Trp
        35                  40                  45

Phe Arg Ser Arg Arg Asn Gln Glu Arg Pro Ser Arg
    50                  55                  60

<210>  8
<211>  61
<212>  PRT
<213>  human

<400>  8

Gly Val Arg Asp Gln Thr Arg Arg Gln Val Lys Pro Asn Ala Thr Leu
1               5                   10                  15

Glu Lys His Phe Leu Thr Glu Gly His Arg Pro Lys Glu Pro Gln Leu
            20                  25                  30

Ser Leu Leu Ala Ala Gln Cys Gly Leu Thr Leu Gln Gln Thr Gln Arg
        35                  40                  45

Trp Phe Arg Arg Arg Arg Asn Gln Asp Arg Pro Gln Leu
    50                  55                  60

<210>  9
<211>  61
<212>  PRT
<213>  human

<400>  9

Gly Ile Glu Asp Ser Gly Pro Tyr Gln Ala Gln Pro Asn Ala Ile Leu

1               5                   10                  15

Glu Lys Val Phe Ile Ser Ile Thr Lys Tyr Pro Asp Lys Lys Arg Leu
            20                  25                  30

Glu Gly Leu Ser Lys Gln Leu Asp Trp Asn Val Arg Lys Ile Gln Cys
        35                  40                  45

Trp Phe Arg His Arg Arg Asn Gln Asp Lys Pro Pro Thr
    50                  55                  60

<210>  10
<211>  61
<212>  PRT
<213>  human

<400>  10

Asn Ile Gln Ala Asn Gly Pro Gln Ile Ala Pro Pro Asn Ala Ile Leu
1               5                   10                  15

Glu Lys Val Phe Thr Ala Ile Thr Lys His Pro Asp Glu Lys Arg Leu
            20                  25                  30

Glu Gly Leu Ser Lys Gln Leu Asp Trp Asp Val Arg Ser Ile Gln Arg
        35                  40                  45

Trp Phe Arg Gln Arg Arg Asn Gln Glu Lys Pro Ser Thr
    50                  55                  60

<210>  11
<211>  60
<212>  PRT
<213>  drosophila_melanogaster

<400>  11

Gly Ile Arg Ser Ser Arg Pro Lys Lys Ala Ala Asn Val Pro Ile Leu
1               5                   10                  15

Glu Lys Thr Tyr Ala Lys Ser Thr Arg Leu Asp Lys Lys Lys Leu Val
            20                  25                  30

Pro Leu Ser Lys Gln Thr Asp Met Ser Glu Arg Glu Ile Glu Arg Trp
        35                  40                  45

Trp Arg Leu Arg Arg Ala Gln Asp Lys Pro Ser Thr
    50                  55                  60

<210>  12
<211>  400
<212>  PRT
<213>  drosophila melanogaster

<400>  12

Met Asp Ile Leu Asn Glu Phe Ser Asn Val Phe Trp Ser Thr His Ile
1               5                   10                  15

Trp Leu Pro Pro Asn Thr Thr Trp Ala Asp Ile Ala Pro Gly Ser Arg
            20                  25                  30

Pro Asp Val Val His Ala Asn Tyr Lys Asp Leu Ile Trp Pro Ile Pro
        35                  40                  45

Phe Ala Ala Val Val Met Leu Val Arg Tyr Thr Leu Glu Arg Phe Trp
    50                  55                  60

Ile Ser Pro Val Gly Lys Ser Leu Gly Ile Arg Ser Ser Arg Pro Lys
65                  70                  75                  80

Lys Ala Ala Asn Val Pro Ile Leu Glu Lys Thr Tyr Ala Lys Ser Thr
                85                  90                  95

Arg Leu Asp Lys Lys Lys Leu Val Pro Leu Ser Lys Gln Thr Asp Met
            100                 105                 110

Ser Glu Arg Glu Ile Glu Arg Trp Trp Arg Leu Arg Arg Ala Gln Asp
        115                 120                 125

Lys Pro Ser Thr Leu Val Lys Phe Cys Glu Asn Thr Trp Arg Cys Ile
    130                 135                 140

Tyr Tyr Leu Tyr Ser Phe Ile Phe Gly Val Ile Val Leu Trp Asp Lys
145                 150                 155                 160

Pro Trp Phe Trp Asp Val Lys Ser Cys Trp Tyr Gly Tyr Pro His Gln
                165                 170                 175

Ser Ile Ser Asn Asp Ile Trp Trp Tyr Tyr Met Ile Ser Met Ser Phe
            180                 185                 190

Tyr Trp Ser Leu Thr Gly Thr Gln Phe Phe Asp Val Lys Arg Lys Asp
        195                 200                 205

Phe Trp Gln Met Phe Ile His His Met Val Thr Leu Leu Leu Met Ser
    210                 215                 220

Leu Ser Trp Val Cys Asn Leu His Arg Val Gly Ser Leu Val Leu Val
225                 230                 235                 240

Val His Asp Cys Ala Asp Ile Phe Leu Glu Ala Ala Lys Leu Thr Lys
                245                 250                 255

Tyr Ala Lys Tyr Gln Lys Leu Cys Asp Ala Ile Phe Ala Ile Phe Thr
            260                 265                 270

Val Val Trp Ile Val Thr Arg Leu Gly Phe Tyr Pro Arg Ile Ile Tyr
        275                 280                 285

Ser Ser Ser Val Glu Ala Pro Arg Ile Leu Pro Met Phe Pro Ala Tyr
    290                 295                 300

Tyr Ile Phe Asn Ser Leu Leu Leu Met Leu Leu Val Leu His Val Ile
305                 310                 315                 320

Trp Thr Tyr Met Ile Leu Lys Ile Val Val Asp Ser Leu Gln Lys Gly
                325                 330                 335

Leu Met Ser Gly Asp Ile Arg Ser Ser Asp Ser Glu Asp Leu Thr Asp
            340                 345                 350

Ser Ser Gly Asn Ala Arg Leu Thr Asn Gly Ser Ala Arg Ser Lys Asn
        355                 360                 365

Lys Ser Ile Ser Ser Ala Pro Ser Asp Lys Gly Ser Ala Gly Gly Ala
    370                 375                 380

Gly Ser Arg Ala Gly Ala Arg Val Ala Thr Thr Glu Arg Arg Glu Glu
385                 390                 395                 400

<210>  13
<211>  2079
<212>  DNA
<213>  drosophila melanogaster

<400>  13
cgcaagttaa cgagacaaga caacaacaat tcgattcggc ggtttttgtt tttttgccag      60

tgtgtgtttg tgctacgctg tgagtgtgca ttagtgtccg gtgctcctgt tgttgttctt     120

gttgctgttg ttgtcgctta ttgttgttct tgctgttatt gttgctgttg ttcgcaaggc     180

acaataacac aggctcatac aattgcgaat atttagaaaa agcaaagcat atacatatgt     240

gacaacaaac gccaaggaaa aactggaaag ctaagcaaag gaaaaccaag aaaaagaaaa     300

caaacaattg agaaattaag aagcggcaac tgaagcagca acatggacat attgaatgag     360

ttcagcaatg tattctggag tacgcacatt tggctgccgc cgaacacaac ctgggcggac     420

attgcgcccg gctcgcgtcc agatgtggtg cacgccaact acaaggacct catatggccc     480

attccattcg ccgctgttgt catgctggtc cgctacacgc tggagcggta agttttccac     540

gtccattttc ccccctccgc actcttccca accaactgac tgactccctt tccattctcg     600

ccattcccgg caacatgatt ccattccgcc tctttctcta accccattca tacggctttc     660

cctcgctctc tcccgctctt tctcgctctc cttaccattg agtaagccca ccgccccacg     720

tttgttgcac catgtacagt aaactttatt cataattcct tcttttctcc atttcatttc     780

attttttttt tatcgacaaa cataacctca cgtggaaacg cccaacattc tgcaagcgtc     840

cgtgtaaata tatatatata tgtgtgtgtg tgtgaaacag cgcttgtttg cttgtgtgcg     900

gtttgtgcgg catacacgcg ttttttgttg ctgcaactcg aagttttccc tttccaagcg 960

acgccggctg cagtttccag ccagccggca acgaatttcc cacaaaatat tagccccact 1020

ccatcttcaa gttcctacta attgtttagt caaaggggaa acatacatac atatatatat 1080

gcacatacat atatatttgt ttatagtcag ctccatgcgg ttttttttta tgattgtatg 1140

gcccgctgag aaattgggat caaaactggc acagtggggc aaatacatag ttaatcaatt 1200

ggccaattgt aggaagtatt tttcataaat atatccaagc caaaaaacgg atatttattg 1260

acttgtcaat aataagaaag tcaacgatat gaaaaccatg tttgttcaga ctttttaacg 1320

acttcaagga aaaccactgc tgcatttgca cagtgcgaga taactaagtt tcgtacagga 1380

aaatccatat tcacaaatat tttattgcaa ttaaagtgcg aaaatgtttt gaaatgccaa 1440

atttccacgc ctgtttgacc cactgtgagc cgctggcatt ttggtcgctt ttcaggccaa 1500

ttaaccagcg cgtcgcattt ggcttcaact atgcatacat acatacatac atacgtaaat 1560

atatggctgt gaatattcga acgagcggca aagttcgtgt gtgcggttcg cgtgcactgg 1620

catttgcatg tggcggagtt gtaattctca tattttgcaa tgtttttggc tgcccttaat 1680

ttgacataaa ttgccagcag caagtccagc ggactggagt ccaaattctg agcatcatta 1740

tcatcatcat catctgccct gcaggacagt gacagtgaca gtgctgttga gcctcgctaa 1800

tatcgccagt tatctaatcc aaattccaaa atattttaac ctgacctaag ctgtgggttc 1860

atcttataga attcgaactg tgactcttgt tttcattcca ttttcagctc tttttcgtcg 1920

acttttttgc attcacgttt gaaaatgaaa ttgagacaaa aaccaaatct gtttcatggc 1980

ggaccacttt ggttattttt tttcttttct tttttttttt tttgtatttc tctgcttctt 2040

aaacgagaat tggtcttcaa ggtcgaattg gggttttgc 2079

<210> 14
<211> 35
<212> DNA
<213> artificial

<220>
<223> primer

<400> 14
cgggatccca acatggacat attgaatgag ttcag 35

<210> 15
<211> 64
<212> DNA
<213> artificial

<220>
<223> primer

<400> 15
gctctagatt aagcgtaatc tggaacatcg tatgggtact cctctctccg ttccgttgtc 60

gcaa 64

<210> 16

<211>  67
<212>  DNA
<213>  artificial

<220>
<223>  primer

<400>  16
ggaattcaac atggactacc catacgatgt tccagattac gctatattga atgagttcag      60

caatgta                                                                67


<210>  17
<211>  62
<212>  DNA
<213>  artificial

<220>
<223>  primer

<400>  17
tggatatcgt acccatacga tgttccagat tacgctcccg taggcaaatc acttggcata      60

cg                                                                     62


<210>  18
<211>  71
<212>  DNA
<213>  artificial

<220>
<223>  primer

<400>  18
ccgctcgagc ggttacagat cctcttcaga gatgagtttc tgctcctcct ctctccgttc      60

cgttgtcgca a                                                           71


<210>  19
<211>  30
<212>  DNA
<213>  artificial

<220>
<223>  primer

<400>  19
ccgctcgagc tactcctctc tccgttccgt                                       30


<210>  20
<211>  56
<212>  DNA
<213>  artificial

<220>
<223>  primer

<400>  20
cttttaatac gactcactat agggagacgt ttctggatat cgcccgtagg caaatc          56


<210>  21
<211>  56
<212>  DNA

<213>  artificial

<220>
<223>  primer

<400>  21
gttttaatac gactcactat agggagacag acccttctgc agagagtcga caacaa          56


<210>  22
<211>  13
<212>  PRT
<213>  artificial

<220>
<223>  oligopeptide

<400>  22

Arg Ser Ser Arg Pro Lys Lys Ala Ala Asn Val Pro Ile
1               5                   10


<210>  23
<211>  15
<212>  PRT
<213>  artificial

<220>
<223>  oligopeptide

<400>  23

Ser Arg Ala Gly Ala Arg Val Ala Thr Thr Glu Arg Arg Glu Glu
1               5                   10                  15


<210>  24
<211>  21
<212>  DNA
<213>  artificial

<220>
<223>  primer

<400>  24
gtgcaccgca agtgcttcta a                                                21


<210>  25
<211>  21
<212>  DNA
<213>  artificial

<220>
<223>  primer

<400>  25
tgctgcactc caaacttcca c                                                21


<210>  26
<211>  20
<212>  DNA
<213>  artificial

<220>
<223>  primer

<400>  26
gctaagctgt cgcacaaatg                                                    20


<210>  27
<211>  20
<212>  DNA
<213>  artificial

<220>
<223>  primer

<400>  27
gttcgatccg taaccgatgt                                                    20


<210>  28
<211>  20
<212>  DNA
<213>  artificial

<220>
<223>  primer

<400>  28
gaatgcacgt ctgaccaatg                                                    20


<210>  29
<211>  21
<212>  DNA
<213>  artificial

<220>
<223>  primer

<400>  29
ttgtcgcaac tcttgctcct g                                                  21


<210>  30
<211>  22
<212>  DNA
<213>  artificial

<220>
<223>  primer

<400>  30
tgagtacggc agctacttcc ct                                                 22


<210>  31
<211>  19
<212>  DNA
<213>  artificial

<220>
<223>  primer

<400>  31
tcaacttgcg gacatcgct                                                     19


<210>  32
<211>  21
<212>  DNA

<213>  artificial

<220>
<223>  primer

<400>  32
acgcacagca gcgacatgta t                                                21


<210>  33
<211>  21
<212>  DNA
<213>  artificial

<220>
<223>  primer

<400>  33
cttttcgctt tgctacgagc c                                                21


<210>  34
<211>  21
<212>  DNA
<213>  artificial

<220>
<223>  primer

<400>  34
ggtcctggaa ccttttcga g                                                 21


<210>  35
<211>  19
<212>  DNA
<213>  artificial

<220>
<223>  primer

<400>  35
ttgcacgaag cggaagatc                                                   19


<210>  36
<211>  20
<212>  DNA
<213>  artificial

<220>
<223>  primer

<400>  36
ctgagtatgg tgtgcgagga                                                  20


<210>  37
<211>  20
<212>  DNA
<213>  artificial

<220>
<223>  primer

<400>  37
gcggttccga tatcgagtta                                                  20

<210>  38
<211>  19
<212>  DNA
<213>  artificial

<220>
<223>  primer

<400>  38
aacagtggcg gattcggtt                                                    19


<210>  39
<211>  21
<212>  DNA
<213>  artificial

<220>
<223>  primer

<400>  39
tactcggagc attggaggca t                                                 21


<210>  40
<211>  31
<212>  DNA
<213>  artificial

<220>
<223>  primer

<400>  40
cgggatcctg atatcgcccg taggcaaatc a                                      31


<210>  41
<211>  37
<212>  DNA
<213>  artificial

<220>
<223>  primer

<400>  41
ataagaatgc ggccgcgatg caacgccacg tgttctc                                37

## Description

The present invention relates in a first aspect to polypeptides useful for the prevention or treatment of various diseases. In particular, the present invention relates to a polypeptide containing a homeodomain for the prevention or treatment of lipid-based metabolic diseases, cancer, (neuro)degeneration, autoimmunity, sepsis, cystic fibrosis, diabetes, or for modulating the immune system in an individual or as an anti aging compound. In another aspect, the present invention relates to nucleic acids encoding said polypeptides, vectors containing said nucleic acid molecules as well as host cells comprising said vector. Further, the present invention concerns isolated antisense RNA or DNA molecules. In addition, the present invention provides methods for screening compounds for treating or preventing said diseases based on the ability to alter expression of ceramide synthases and its cleavage.

### Prior art

Energy homeostasis is critical for growth and development. It depends on the ability to control the balance between lipid synthesis and storage and lipid mobilization during times of energy abundance or deprivation, respectively. Storage fat is deposited as triacylglycerols (TAGs) in intracellular lipid droplets which accumulate in specialized organs such as mammalian adipose tissue or the fat body of flies. Starvation-induced lipolysis of TAGs is induced by lipases and leads to the release of free fatty acids (FA) or sn-1,2-diacylglycerol in mammals and insects, respectively, into the circulatory system. In insects, these lipids are carried by the lipoprotein lipophorin for delivery to tissues, where it is hydrolyzed to fatty acids. Chronic dysregulation of the balance between lipolysis and lipogenesis may lead to metabolic abnormalities such as obesity, lipodystrophy syndromes or insulin resistance in humans.

The regulation of the lipid metabolism is particularly important during growth. On the cellular level, sterols in mammalian cells or phosphatidylethanolamine, the major phospholipids in *Drosophila,* control the release of sterol regulatory element binding protein (SREBP) from cell membranes, exerting feedback control on the synthesis of fatty acids and phospholipids. SREBPs are membrane bound transcription factors that monitor cell membrane composition and adjust lipid synthesis accordingly. The *de novo* synthesis of sphingolipids is also pivotal since sphingolipids are essential structural components of eukaryotic membranes and play important roles as second messengers regulating apoptosis, survival and differentiation. Misregulation of the sphingolipid metabolism is involved in the etiology and pathology of a number of human diseases, including cancer, immunity, cystic fibrosis, emphysema, diabetes, and sepsis. The enzymes of the sphingolipid pathway are conserved in all genetically studied eukaryotes. However, *in vivo* information on how these enzymes are regulated in response to growth requirements or during starvation are either limited by early lethality of the knock out animals reflecting the fundamental necessity of these enzymes or by the lack of mutants. The latter includes mutants for key enzymes of the pathway such as (dihydro)ceramide synthases, which produce the precursor metabolites for all sphingolipids.

Lipid and carbohydrate metabolism on the cellular and organismic level is regulated by antagonistic activities of insulin and glucagons in mammals or insulin-like peptides and the glucagons-like adipokinetic hormones (Akhs) in insects. Whereas insulin-like peptides promote cellular glucose import and energy storage in the form of glycogen and TAGs, glucagons or Akh increase lipolysis and glycogenolysis during energy-requiring activities. In flies, insulin-like peptides are generated in small clusters of cells in the brain, the insulin-producing cells (IPCs), as well as in the gut and imaginal discs. Akh is produced in the corpora cardiaca (CC) of the ring gland, which is a neuroendocrine gland associated with the brain. Like mammalian glucagons activity in liver, Akh has been shown to interact with a G-protein-coupled transmembrane receptor inducing TAG-lipase activities in the fat body, including brummer, which is an evolutionary conserved adipose triglycerides lipase (ATGL) localized to lipid droplets. However, mechanisms by which cellular lipid metabolism might be interlaced with hormone-dependent body fat regulation are unknown at present.

(Dihydro)ceramide synthases (Lass/CerS) utilize long-chain bases, sphinganine or sphingosine, and FA-CoAs with varying chain length to produce (dihydro)ceramide. Ceramides are structural components of all cellular membranes and can act as signalling molecules in cell growth, differentiation and apoptosis. Lass/CerS family members contain five or six transmembrane domains, a catalytic Lag1 motif and an amino-terminal domain showing sequence homology to DNA-binding homeodomains (Hox domain) (Gehring et al., 1994, Ann Rev Biochem., 63, 487-526). The lag1 motif of Lass/CerS proteins, which are found in organisms ranging from yeast to mammals, is functionally required for (dihydro)ceramide synthesis and is contained within a stretch of 52 amino acids (Pewzner-Jung et al., 2006, J. Biol. Chem., 281, 25001-25005).

Pewzner-Jung et al., supra, describe that a subset of Lass genes contain said homeobox or Hox domain. In humans and mice, all but Lass1 have a Hox domain while in Drosophila only one of the four TLC (Tramm-Leg-CLN 8 domain) have a Hox domain, and no Hox domains are found in yeast and plant Lass genes. It is identified in Pewzner-Jung et al. and also in Mesika. A., et al (J. Biol. Chem, 2007, 282, 27366-27373) that the Hox domain is not involved in the catalytic mechanism of ceramide synthases and the function of the Hox domain remains unknown.

Today it is known that ceramide synthases are involved in anti-aging and cell proliferation.

The present invention provides homologues of the Lass molecules as well as new fragments thereof displaying new functions in lipid metabolism. In addition, the present invention provides polypeptides useful for the prevention or treatment of lipid-based metabolic diseases, cancer, (neuro)degeneration, autoimmunity, sepsis, cystic fibrosis, diabetes, modulating the immune system in an individual or as an anti aging compound.

### Summary of the invention

The present invention is directed to a polypeptide comprising the sequence of Seq ID. No. 1 for the prevention or treatment of various types of diseases.

Preferably, said polypeptide comprises a sequence of Seq. ID No. 2, and, more preferably, does not contain Seq. ID No. 3 or a functional fragment thereof of at least six amino acids having ceramide synthase activity. In particular, the polypeptide according to the present invention is a polypeptide of Seq. ID Nos. 4 or 5, like any one of Seq ID. Nos. 6 to 11.

In another embodiment, a polypeptide is provided containing a sequence of Seq. ID No. 1 or Seq. ID No. 2, and, in addition, of Seq. ID No. 3 **characterized in that** said sequence does not contain a restriction site allowing restriction of the ceramide synthase protein in two fragments whereby the N-terminal fragment comprises the homeodomain and the C-terminal fragment comprising the Lag domain

Further, nucleic acid molecules encoding the polypeptides according to the present invention, vectors and host cells containing the same as well as isolated antisense RNA or DNA, silencer mRNA, RNAi, microRNA, or non-coding RNA complementary to selectively hybridize to the nucleic acid molecules according to the present invention are provided.

A further embodiment of the present invention provides a method for screening compounds for preventing or treating several types of diseases, said compounds are characterized in altering the expression of a ceramide synthase of fragments thereof, altering posttranslational modification of said ceramide synthase or altering the presence of cleaved fragments containing the homeodomain which are part of the polypeptide having ceramide synthase activity in cells incubated with the compounds to be tested compared to the cells incubated in the absence of the compound to be tested.

Furthermore, in another embodiment the present invention provides dietary supplements and food containing compounds identified according to the present invention and inhibiting cleavage of a homeodomain containing fragment from a polypeptide having ceramide synthase activity.

Finally, the present invention provides a composition containing the polypeptide according to the present invention not having ceramide synthase activity for systemic, particular topical administration to a subject.

### Brief description of the drawings

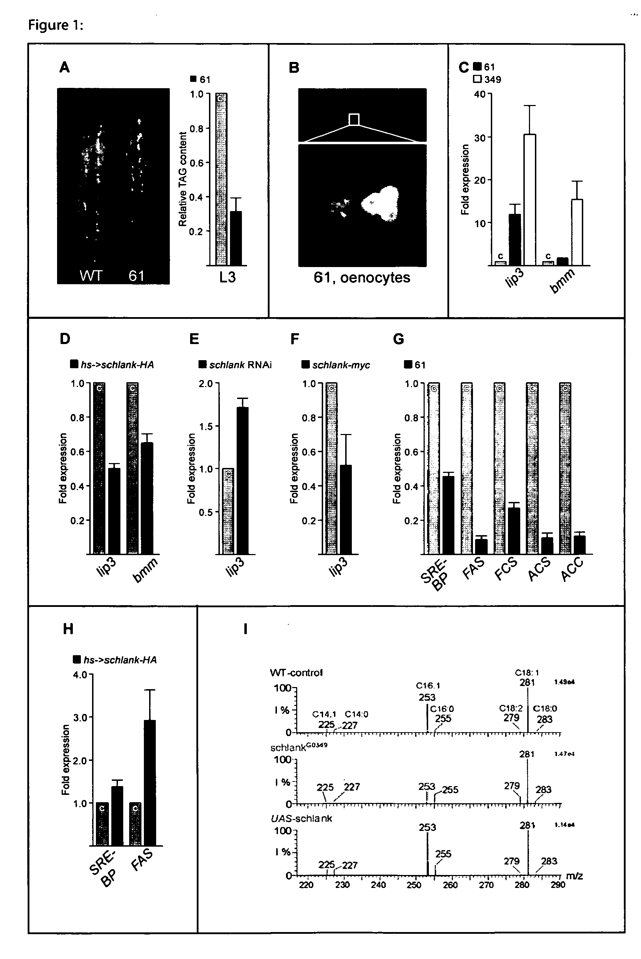

Figure 1: **schlank controls the balance between lipolysis and lipogenesis. (A)** *schlank*^{G0061}(61) or *schlank* RNAi knock down larvae are thinner than WT larvae\ when reaching the same size at L3 stage. Triacylglycerol (TAG) level of *schlank*^{G0061} (black bar) compared to WT in third instar wandering stage larvae. **(B)** Oenocytes are present in *schlank*^{G0061} larvae. **(C)** Transcript levels of *lip3* and *bmm* lipases are upregulated in *schlank^{G0061}* (third instar, black bars) and *schlank^{G0349}* (48h AEL, white bars) mutants compared to corresponding WT larvae. **(D)** Overexpression of UAS-*schlank-HA* by *hs*-Gal4 (black bars) decreases *lip3* and *bmm* transcript levels. **(E)** *schlank* RNAi in SL2 cells (black bar) increases the *lip3* level. **(F)** *lip3* expression in SL2 cells overexpressing *schlank-myc* (black bar). **(G)** Transcription of *dSREBP* (*SREBP*) and its downstream targets fatty acid synthase (FAS), Fatty acyl-CoA synthetase (*FCS*), acetyl-CoA synthetase (ACS) as well as acetyl-CoA carboxylase (*ACC*) in *schlank^{G0061}* (black bars) compared to WT. **(H)** *dSREBP* and FAS transcript level in larvae overexpressing *schlank-HA.* **(I)** Analysis and quantification of fatty acids in WT, *schlank^{G0349}* mutants, and in larvae overexpressing *schlank* by ESI-MS (for further details, see Supplementary Information). **(A-I)** Grey bars (marked by c) indicate WT control, error bars SEM.

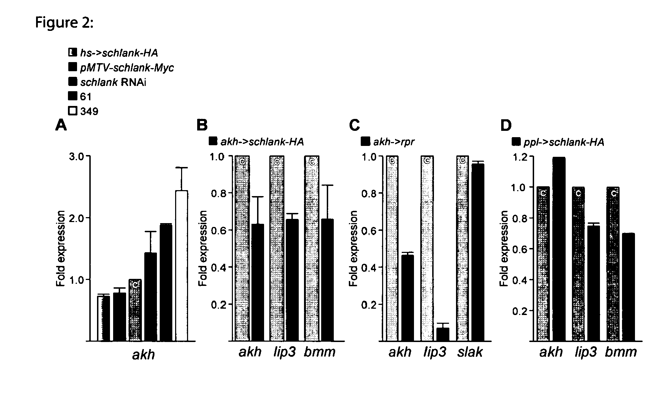

Figure 2: ***schlank* regulates transcript levels of the lipolytic *akh* and of *lipase* genes. (A)** *akh* mRNA levels when UAS-*schlank-HA* is overexpressed in larvae by heat shock, when pMTN5-*schlank-Myc* is expressed in SL2 cells and when *schlank is* knocked down by RNAi; *akh* mRNA levels in *schlank^{G0061}* and in *schlank^{G0349}* compared to WT controls (grey bar marked by c). **(B)** Changes in *akh, lip3* and *bmm* transcript levels in larvae overexpressing *schlank-HA* using *akh*-Gal4 (black bars). **(C)** Ablation of Akh-expressing ring gland cells using *akh*-Gal4 in combination with UAS-reaper (black bars) leads to decreased akh and *lip3* transcripts, but has no effect on *schlank* expression. **(D)** Overexpression of *schlank-HA* in the fat body by *pp*/*-*Gal4 (black bars) decreases *lip3,* but not *akh* transcripts. **(A-D)** WT controls (marked by c) are indicated by grey bars, error bars show SEM

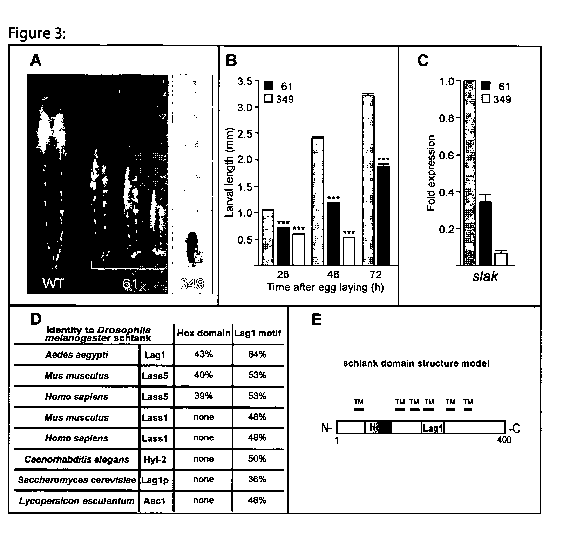

Figur 3: ***schlank* is essential for larval growth. (A)** *schlank^{G0061}* (61) and *schlank^{G0349}* (349) larvae compared to wild type (WT) larvae 72h after egg laying (AEL). Note the growth variance in *schlank^{G0061}*. **(B)** Average length 24-25h, 48-49h and 72-73h AEL of WT (n=123, n=81, n=48), 61 (n=54, n=187, n=63) and 349 (n=14, n=106) larvae. **(C)** Reduced *schlank* transcription in *schlank* mutants. **(D)** *Drosophila* schlank protein domains compared to other Lass/CerS family members. Amino acid sequence identity is indicated in %. **(E)** Predicted putative domain structure of *Drosophila* schlank. Transmembrane domain (TM) according to TMpred (http://www.ch.embnet.org/software/TMPRED_form.html). **(B-C),** Grey bars (for realtime additionally marked by 'c') indicate wild type, black bars *schlank^{G0061}* and white bars *schlank^{G0349}.* Asterisks in (B) show significant differences to wild type (p<0.001). Error bars indicate SEM.

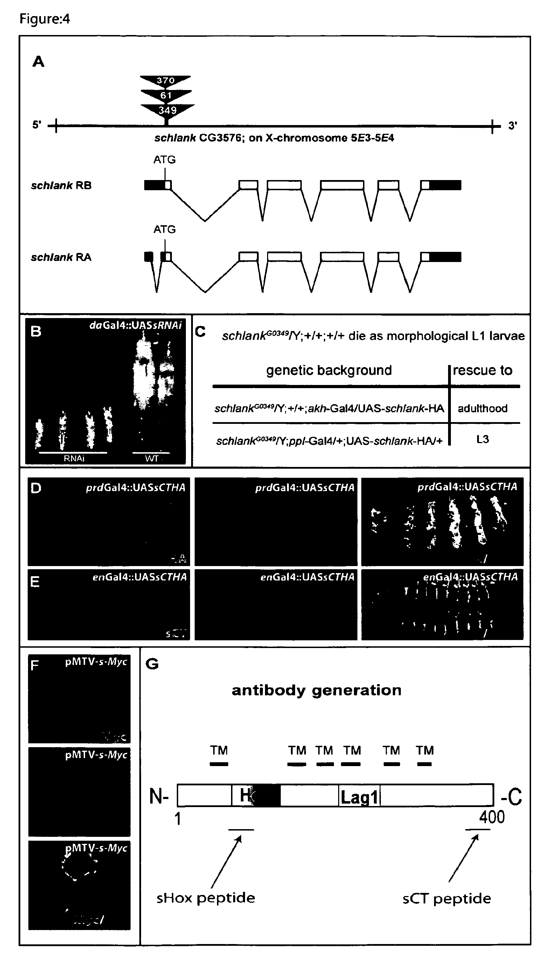

Figure 4 ***schlank* genomic locus and schlank antibody specificity. (A)** Molecular organization of the *schlank* transcription unit and localization of the P-insertions (see Materials and methods). *schlank* encodes a predicted protein of 400 amino acids with a predicted molecular weight of 46.3 kDa. **(B)** Phenocopy of growth defects using a *schlank* RNAi construct (w1118; pMF3 DLAG1 RNAi #33896) in combination with a da-GAL4 driver line. Comparison of larvae of the same developmental stage **(C)** Tissue specific rescue of *schlank^{G0349}* mutants by reconstituting *schlank-HA* in the ring gland and the fat body using *akh*-Gal4 and *ppl-*Gal4, respectively. **(D)** Ectopic expression of UAS-*schlank-CT-HA* (*UASsCTHA*) in stripes using the *paired-Gal4* driver. The anti-sHox antibody (red) detects ectopic schlank-HA expression in a *paired* like pattern and thereby colocalizes with the anti-HA antibody (green). **(E)** Expression of UAS-*schlank* using *engrailed (en)*-Gal4 in combination with UAS-*schlank* results in a specific expression of schlank in an *en-*like expression pattern. The staining pattern of sCT (green) and sHox (red) is completely overlapping further confirming the specificity of the antibodies used. The en-like expression pattern was lost, when these embryos were incubated with excess of sCT or sHox peptide (data not shown). **(F)** SL2 cells expressing pMTV-*schlank-Myc (pMTV-s-Myc)* stained with anti-Myc (green) and sCT (red). The staining pattern of sCT is overlapping with the anti-Myc signal further confirming the specificity of the sCT antibody. **(G)** Putative structure of schlank. The localization of the schlank peptides used for antibody production are indicated by arrows.

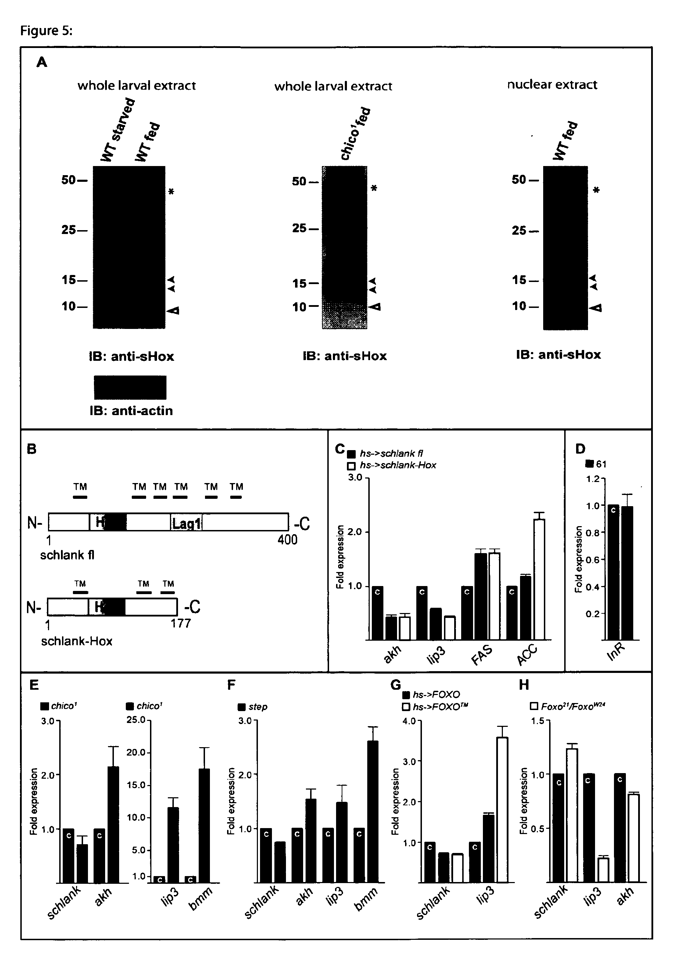

**Figur 5**: Processing of schlank protein in response to nutrient abundance and negative regulation of *schlank* by the insulin-responsive dFOXO transcription factor. (A) Western immunoblot analysis of schlank using the sHox antibody. The source of each lysate is indicated above the immunoblots. Whole cell larval extracts from well fed and starved early third instar wild type larvae. The blot displays signals corresponding to full-length (asterisk) and several smaller Hox domain-containing, 26 kDa, 15 kDa (arrow) and 10 kDa polypeptides (triangle) in fed larvae. In extracts of starved larvae, the 10 kDa polypeptide is absent. Actin was used as loading control. In extracts of the well fed insulin receptor substrate mutant *chico¹*, the 15 kDa polypeptides (arrow) are reduced and the 10 kDa polypeptide (triangle) is absent. The 15 kDa and the 10 kDa polypeptides are detected in nuclear extracts of well fed larvae. (B) N-terminal 177 amino acid schlank-Hox construct without the highly 25 conserved catalytic Lag1 motif. (C) *akh* and *lip3* mRNA levels are negatively, *FAS* and ACC are positively regulated in larvae overexpressing *schlank*-Hox or full length (fl) *schlank.* (D) Insulin signalling is not affected in *schlank^{G0061}* larvae (black bars), as indicated by unaltered insulin receptor mRNA expression, as compared to the WT control larvae (c). (E) *schlank* expression is reduced in *chico¹* mutants (black bars), resulting in an elevation of *akh* as well as *lip3* and *bmm* mRNA levels (shown in an adjacent graph due to higher expression levels). (F) *schlank* mRNA levels are reduced in *steppke* mutants (black bars, *step*), schlank target genes *akh*, *lip3* and *bmm* are upregulated. (G) *schlank* mRNA levels are reduced and *lip3* expression is increased in larvae overexpressing *FOXO* (black bars) or *FOXO*^{™} (white bars) by heat shock. (H) *schlank* and *akh*/*lip3* mRNA levels increase and decrease, respectively, in starved *FOXO²¹*/*FOXO^{W24}* (white) mutants compared to starved WT larvae (grey bar). (D-H) Error bars indicate SEM.

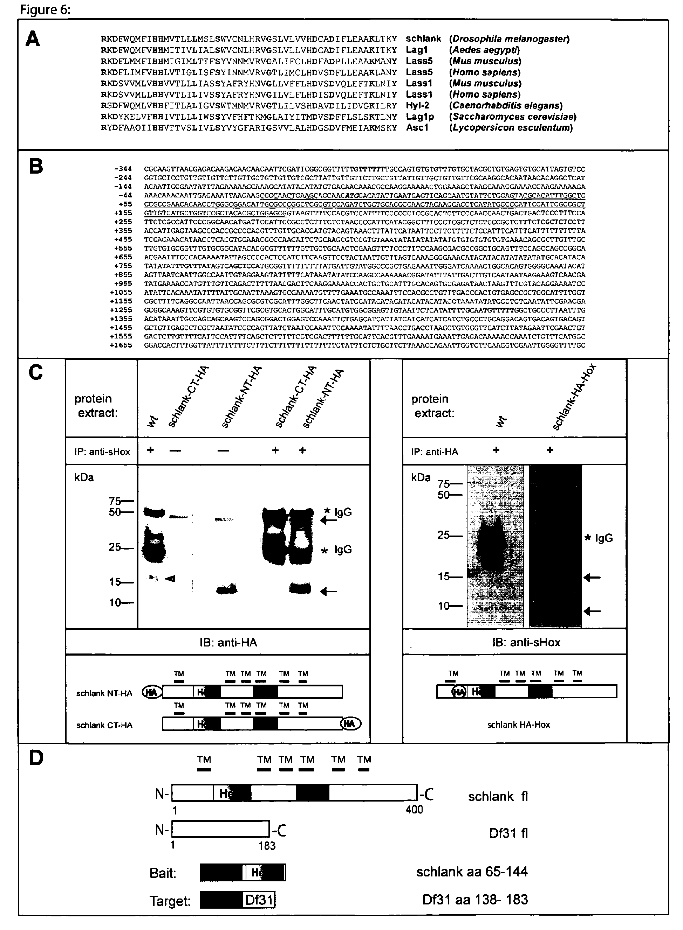

Figur 6: **Conserved motifs of the schlank protein, its putative regulatory region, immunoprecipitation of HA-tagged schlank fusion proteins, and a yeast two hybrid screen. (A)** schlank histidines (marked) is highly conserved in all Lag1 paralogs and has been shown to be essential for (dihydro)ceramide synthesis in Lass/CerS family members. Other highly conserved residues are marked in addition. (B) Putative dFOXO binding sites in the putative *schlank* regulatory genomic regions. Indicated are forkhead responsive elements and common core forkhead binding sequences, repsectively. (C) Proteolytic processing of schlank at the N-terminus. Immunoprecipitation (IP) and immunoblot (IB) of protein lysates from larvae expressing HA-tagged schlank fusion proteins. The source of the lysates is indicated above the immunoblots and schemes of tagged fusion proteins are depicted underneath the immunoblots. Immunoprecipitation using either schlank-NT-HA or schlank-HA-Hox lysates indicates that schlank is processed at the N-terminus, which results in the release of a 15 kDa polypeptide (left panel, 15 kDa band, arrow) and finally in the release of a peptide fragment containing the Hox domain (right panel, 10 kDa band, arrow). The size corresponding to the full length schlank protein, the N-terminally derived 15 kDa polypeptide and to the 10 kDa Hox domain containing polypeptide is indicated by an arrow. No specific bands are detected using protein extract of wild type larvae not expressing HA-tagged schlank fusion proteins after the application of heat shock. Unspecific bands are marked with a triangle (Δ). Bands corresponding to the IgG light and heavy chain s are marked with an asterisk (*). (D) Yeast two hybrid screen using the schlank Hox domain as a bait to screen a 0- to 24-h embryonic *Drosophila* library. Out of 31 positive clones three independent Df31 clones were identified. All three Df31 clones were in frame with the myristilation signal (Mys). Df31 full length protein (Df31fl) and schlank full length protein (schlank fl).

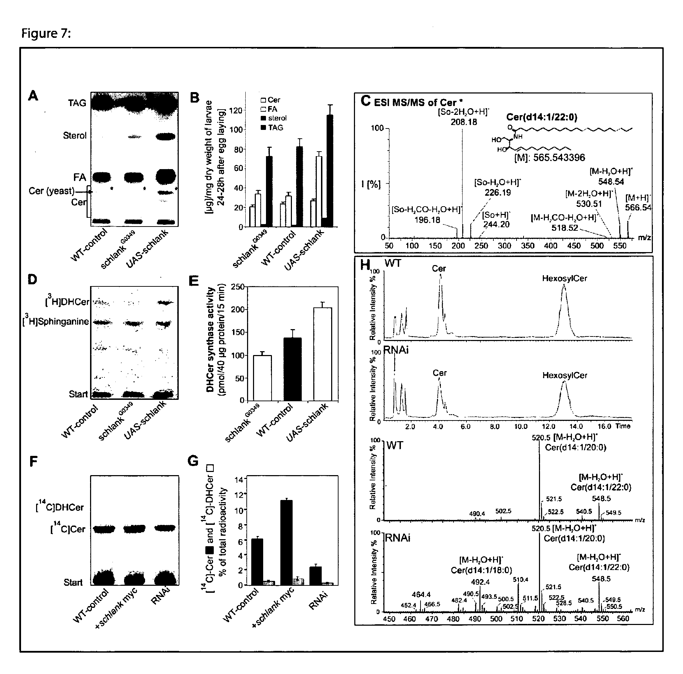

**Figure 7****. schlank regulates lipid metabolism. (A-B)** Ceramides (Cer), fatty acids (FA), as well as triacylglycerols (TAG) are increased in larvae overexpressing *schlank* (UAS-*schlank*), whereas the Cer and TAG levels are reduced in *schlank^{G0349}* mutants (note that these mutants still contain maternally provided Schlank). Phytoceramide Cer(tC18:0/C26 or tC20:0/C26) originates from food (yeast). **(A, C)** To determine the sphingoid base and fatty acid composition, individual ceramide fractions (i.e. see asterisk*) were identified by ESI-MS/MS (So: sphingosine base). Overexpressing *schlank* enhances dihydroceramide (DHCer) synthesis in larvae (D, E) and in cultured SL2 cells **(F, G).** Dihydroceramide synthesis is reduced in *schlankG0349* mutants **(D, E)** and in RNAi SL2 cells **(F, G, H).** Further semiquantitative analysis by LC/APCI-MS of wt and RNAi SL2 proofs a significant downregulation of ceramides in schlank RNAi cells (H, upper panels, data in chromatograms are normalized to fixed intensity) and qualitative analysis of the mass spectra shows that Cer(d14:1/20:0) and Cer(d14:1/22:0) are the most abundant species (H, lower panels, note that the data in the mass spectra are normalized to the largest peak on display (m/ z= 520.5)).

### Detailed description of the invention

The present invention is directed in a first aspect to a polypeptide comprising the sequence of Seq. ID No. 1
RRX₁QX₂P (Seq. ID No.1)
wherein X₁ and X₂ are any amino acid,
for the prevention or treatment of lipid-based metabolic diseases, cancer, (neuro)degeneration, autoimmunity, sepsis, cystic fibrosis, diabetes, modulating the immune system in an individual or as an anti aging compound.

In a preferred embodiment, X₁ is an amino acid of A or N. Furthermore, preferably, X₂ is an amino acid having a basic side chain, in particular, X₂ is R or K.

Preferably, said polypeptide according to the present invention comprises the sequence of Seq. ID No. 2
WX₃RX₄RRX₁QX₂P (Seq ID No. 2)
wherein X₁, X₂, X₃ and X₄ are any amino acid, or a functional fragment thereof of at least six consecutive amino acids.

Preferably, X₁ is an amino acid of A or N. Furthermore, preferably, X₂ is an amino acid having a basic side chain, in particular, X₂ is R or K. Moreover, X₃ is preferably any one of an amino acid having an aromatic moiety, in particular, W or F. Further, X₄ is preferably any one of L, H, S, R, or Q.

Further preferred, the polypeptide according to the present invention is a polypeptide comprising a sequence of Seq. ID. No. 4 or 5, respectively.
LE(X)ₙ WX₃RX₄RRX₁QX₂P (Seq ID. No. 4 and 5)
wherein X₁, X₂, X₃, and X₄ are defined as above, X is any amino acid residue and n is an integer of 30 (Seq. ID. No.4) or 31 (Seq. ID. No.5), preferably 31.

Moreover, in a particular preferred embodiment the polypeptide according to the present invention is a polypeptide comprising either Seq. ID No. 1 or Seq. ID No. 2 or a functional fragment thereof and, in addition, does not contain Seq. ID No 3. or a functional fragment thereof of at least six consecutive amino acids having a ceramid synthase activity. Seq. ID No.3 comprises the Lag domain of ceramide synthase enzyme of the schlank molecule.

When referring to the peptide of Seq. ID. No. 3 and functional derivatives thereof, this term includes the human analogs of the Lag domain of Seq. ID. No.3 of drosophila melanogaster, in particular the Lag domain present in the Lass proteins in human or mice.

Particularly preferred the polypeptide according to the present invention is a polypeptide of Seq. ID Nos. 6 to 11 or fragments thereof comprising at least the sequences of either Seq ID. No 1, 2 or 4 or 5, respectively.

In this connection, the term "any amino acid" refers to amino acids residues naturally occuring in peptides or proteins including the 20 amino acids encoded by the genetic code as well as modified forms thereof.

Seq. ID No. 1 represents the minimal consensus sequence of a polypeptide encoding at least parts of the homeodomain or Hox domain. The Hox domain is derived from homeobox proteins, which are sequence specific transcription factors important in development. Mammalian and, as identified herein, *Drosophila* ceramide synthase proteins contain a number of distinguishing features prominent among these are two domains, namely i) the Lag domain, a region of about 50 amino acids residues and ii) the homeodomain or Hox domain which is found in all CerS genes apart from CerS 1 or Lass 1. Said Hox domain is located N-terminally of the Lag domain.

When searching for genes controlling larval growth in drosophila the inventors identified three P element alleles of a novel focus on the x chromosome, which they further refer to as schlank. Sequence analysis indicated that the schlank gene (Seq. ID. No. 13) encode a transmembrane protein (Seq. ID. No. 12) with high homology to members of the conserved non longevity assurance homologue Lass/CerS family of (dihydro)ceramide synthases.

The schlank gene and the polypeptide encoded by said schlank gene, Schlank, contains a homeodomain unit and a Lag domain unit as distinguishing features of ceramide synthase proteins.

In the following, the term "homeodomain" or "Hox domain" refers to a DNA sequence (of about 180 base pairs) contained in a gene (e.g. hox gene), and code for a protein domain (of about 60 amino acids) that is highly conserved, and can bind to DNA to control gene expression.

The homeobox domain was first identified in a number of drosophila homeotic and segmentation proteins, but is now known to be well-conserved in many other animals, including vertebrates (PUBMED:2568852), (PUBMED:1357790). Hox genes encode homeodomain-containing transcriptional regulators that operate differential genetic programs along the anterior-posterior axis of animal bodies (PUBMED:12445403). The domain binds DNA through a helix-turn-helix (HTH) structure. The HTH motif is characterised by two alpha-helices, which make intimate contacts with the DNA and are joined by a short turn. The second helix binds to DNA via a number of hydrogen bonds and hydrophobic interactions, which occur between specific side chains and the exposed bases and thymine methyl groups within the major groove of the DNA. The first helix helps to stabilize the structure (SMART accession number SM00389).

Furthermore, the term "lipid-based metabolic diseases" refers to diseases including but not restricted to hyperlipidemia, insulin resistance, diabetes, obesity, starvation, coronary artery disease, arteriosclerosis, lipid-metabolism associated myocardial infarction, lipid storage diseases, lipoma, and liposarcoma.

The term "functional fragment" refers to amino acid or nucleic acid molecules displaying the same functional activity as the whole polypeptide or nucleic acid, e.g. having the same enzymatic activity whereby the activity is at least 50% of the activity of the whole polypeptide or the same activity in activating transcriptional processes, e.g. in inducing transcription of specific mRNA, etc.

The term "polypeptide" means, according to the present invention, a peptide(s) or a (poly)peptide(s) which encompass amino acid chains of any length, wherein the amino acid residues are linked by covalent peptide bonds, as long as said polypeptide include the sequence noted in connection with that polypeptide. However, peptidomimetics of such proteins wherein amino acid(s) and/or peptide bond(s) have been replaced by functional analogues are also encompassed by the invention. In accordance with this invention, a protein may comprise different protein species. A protein species is (furthermore and not limiting) defined by a chemical composition and modifications of said peptide(s)/(poly)peptide(s) by, inter alia, glycosylations, acetylation, phosphorylations, lipidations or by amino acid exchanges. Thus, the term "polypeptide" refers to a polypeptide of amino acids and does not refer to a specific length of the product; hence, peptides, oligopeptides and proteins are included within the definition of polypeptides.

It has been recognized that the polypeptide encoded by the schlank gene is processed, thus, resulting in two polypeptide fragments, whereby the N-terminal polypeptide contains the homeodomain while the C-terminal derived polypeptide contains the Lag domain. In addition, it has been recognized that the N-terminal fragment obtained after cleavage of Schlank and related CerS display new functions as described in more detail below and in the experimental part, in particular, said fragment enters the nucleus and induce transcription of various genes involved in lipid homeostasis as well as regulating other factors involved in insulin and sphingolipid metabolism.

Mutants not containing the respective restriction site, thus, resulting in polypeptides not cleavable in a polypeptide fragment containing the homeodomain and a second fragment containing the Lag domain, demonstrated a phenotype of reduced fat uptake. That is, it has been identified that the polypeptide schlank regulates the balance between lipolysis and lipogenesis.

Further, it is has been shown, that schlank controls TAG metabolism by recognizing that schlank larvae showed the same size as WT larvae. However, they appeared much slimmer and somewhat transparent indicating a loss of storage fat in the fat body. A function in regulating fat storage or mobilization has previously not been observed for Lass/CerS family members due to the lack of animal models.

Storage fat is deposited as TAGs in lipid droplets of the fat body. We compared wandering third instar larvae, a discrete developmental stage when feeding has ceased, of the weaker schlank mutant allele. Morphological criteria were used to ensure that wild type and mutant animals of the same developmental stage were compared (Gutierrez et al., 2007, J. Mol. Biol. 373, 903-912). It has been found that TAG levels are markedly decreased in schlank mutants, as compared to wild type animals (Figure 1A). Consistently, the fat body cells contained reduced amounts of lipid droplets, as determined by using the lipid droplet marker Oil Red O and appeared more translucent, as compared to wild type animals (Figure1A; data not shown). Upon fasting, mobilized lipids accumulate in the hepatocyte-like oenocytes, which have a regulatory function on the fat body. The cell number and the morphology of oenocyte cells were normal in schlank mutant larvae (Figure 1 B). In summary, these data indicate a failure of lipogenesis and/or increased lipolysis in schlank mutants.

Lipolysis in response to fasting is accompanied by an upregulation of lipases, the rate limiting enzymes regulating TAG mobilization in insect fat body cells. In schlank mutants, mRNA levels of brummer encoding an ATGL lipase, and of lip3, which encodes a TAG lipase, were strongly increased (Figure 1C). In contrast, the expression of both lipases was decreased upon ubiquitous expression of schlank in larvae using *hs*-Gal4: UAS-*schlank* (Figure 1 D). Knock down of schlank in *Drosophila* SL2 tissue culture cells by RNAi or overexpression of schlank also resulted in a transcriptional upregulation or downregulation, respectively, of *lip3* expression (Figures 1 E and 1 F). The tissue culture experiments confirmed the in vivo data on schlank regulating lipase expression and further ruled out starvation as the primary cause for the increased lipase levels.

Lipid homeostasis is controlled in all metazoans studied by a regulatory pathway involving the sterol regulatory element binding protein (SREBP). SREBPs are transcription factors required for the synthesis of cholesterol and unsaturated FAs in mammals and saturated FAs in *Drosophila.* SREBPs are generated as precursors containing two membrane-spanning helices inserted into ER membranes. In response to cellular lipid needs, a complex of SREBP and an escort factor exits the ER and travels to the Golgi apparatus, where it is subject to sequential cleavages. As a product the transcriptionally active domain is released from the membrane, enabling it to travel to the nucleus and activate the transcription of target genes required for FA synthesis. In schlank mutants, the level of SREBP is markedly decreased (Figure 1 G). Consistently, transcription of the SREBP target genes acetyl-CoA synthetase (ACS), acetyl-CoA carboxylase (ACC), and fatty acid synthase (FAS), encoding enzymes of fatty acid biosynthesis and fatty acyl-CoA synthetase (FCS) encoding an enzyme involved in synthesis of phospholipids, are strongly reduced (Figure 1 G). Similar results were obtained in SL2 cells upon knock down of schlank. In contrast, overexpression of schlank in larvae led to an increase of SREBP transcript levels and of SREBP-dependent target gene expression, including FAS (Figure 1H). Consistently, a downregulation of FA levels (particularly myristic acid (C14:9), myristoleic acid (C14:1) and palmitric acid (C16:1)) in schlank mutant larvae and elevated levels of FAs in larvae overexpressing schlank, has been determined as demonstrated by ESI-MS analysis and quantification of FA (Figures 1I). In summary, these data indicate that TAG mobilization is induced in schlank mutant larvae due to an upregulation of lipase expression. Furthermore, they identify schlank as a positive regulator of SREBP transcript levels and of SREBP-dependent FA and phospholipids synthesis.

The larvae mutant having no cleavage of the schlank polypeptide are slimmer. It has been recognized that depending on the nutritional state of the individual, proteolytic cleavage of the schlank protein occurs.

Thus, inhibiting cleavage of the ceramide synthase comprising the homeodomain and the Lag domain allows adjusting the balance between lipolysis and lipogenesis to lipolysis, hence, reducing fat.

Therefore, in another embodiment of the present invention, a method is provided for screening and/or identifying compounds useful as inhibitors of the cleavage of ceramide synthase. Said inhibitors are *inter alia* particularly useful in dietary supplements and food.

Hence, food and dietary supplements are provided containing an inhibitor of ceramide synthase cleavage, thus, altering the amount of the cleaved homeodomain of a ceramide synthase containing said homeodomain.

In addition, the present invention relates to a method for screening compounds for preventing or treating lipid based metabolic diseases, cancer, neurodegeneration, autoimmunity, sepsis, cystic fibrosis, diabetes or modulating the immune system in an individual or as an anti aging compound comprising the steps of
a) providing a cell line expressing a ceramide synthase containing a homeodomain comprising a sequence of any one of Seq ID Nos.1,2, and 4 to 11;
b) incubating the cells of a) in the presence and absence of compounds to be tested;
c) determining altered expression of the ceramide synthase or fragments thereof, altered posttranslational modification of the ceramide synthase, or the presence of cleaved homeodomain in cells incubated with the compound to be tested compared to the cells incubated in the absence of the compound to be tested.

Ceramide synthases are involved in sphingolipid synthesis. As identified before, the sphingolipid metabolism is involved in the etiology and pathology of a number of diseases, including cancer, immunity, cystic fibrosis, emphysema, diabetes, and sepsis (Lahiri and Futerman, 2007, Cell. Mol. Life Sci. 64, 2270-2284). Phospholipids, namely ceramides, mediate anti-proliferative responses. Thus, increasing the amount of ceramides by elevating the lipogenesis pathway and lipid homeostasis might be beneficial for example for cancer treatment.

Furthermore, ceramides and ceramide metabolism play an important role in other types of diseases, for example neurodegeneration, autoimmunity, sepsis, cystic fibrosis, diabetes or the modulation of the immune system and are acting as anti aging compounds. The present inventors identified that the cleaved polypeptide comprising the homeodomain is able to enter the nucleus and, thus, act as an activator for transcription of molecules involved in lipogenesis, said molecules allow to upregulate sphingolipid synthesis and, as a consequence, allow to initiate the beneficial effect known for said sphingolipid compounds.

Hence, the polypeptides according to the present invention are beneficial for prevention or treatment of lipid-based metabolic diseases, cancer, neurodegeneration, autoimmunity, sepsis, cystic fibrosis, diabetes or modulating the immune system or are acting as an anti aging compound.

It has been shown previously that lipolysis can be induced by Akh-dependent activation of TAG-lipase activities in the fat body. To test whether the upregulation of lipase expression in schlank mutants occurs via a regulatory effect on Akh expression in the corpora cardiaca cells of the ring gland, the akh expression levels in the schlank alleles by using quantitative RT-PCR experiments was tested. It was found that mRNA levels of the akh gene are strongly increased in schlank mutants, consistent with the increase in lipase levels and the mobilization of TAGs observed in the mutants (Figure 2A). Conversely, upon ubiquitous expression of schlank in third instar larvae via heat shock or upon cell-specific overexpression of schlank in Akh-producing cells using the akh-Gal4 driver, a reduction of akh, lip3 and brummer mRNA levels has been determined (Figure 2B). Furthermore, knock down of schlank in SL2 cells using RNAi showed increased akh, lip3 and brummer mRNA levels, whereas expression of the genes was decreased upon overexpression of schlank (not shown). In contrast, expression of schlank is unchanged in animals in which akh expression is strongly reduced due to the induction of apoptosis in the Akh cells, caused by expression of the cell death factor reaper in akh-Gal4:UAS-reaper transgenic flies (Figure 2C). It is concluded from these data that schlank is upstream of akh and a negative regulator of akh expression in the corpora cardiaca cells of the ring gland. To test whether schlank acts indirectly via Akh on the regulation of lipase mRNA levels in the periphery, schlank has been overexpressed specifically in the fat body by using ppl-Gal4 in combination with UAS-schlank. Whereas the akh levels were not reduced, as expected, the mRNA levels of lip3 and brummer were downregulated (Figure 2D). This indicates that schlank not only acts vie Akh on lipase mRNA levels, but also has a direct negative regulatory effect on lipase mRNA levels in the peripheral fat body. Consistently, it has been shown that prominent expression of schlank in the Akh-expressing cells of the corpora cardiaca, and in the fat body occurs. Expressing a schlank-expressing transgene exclusively in the akh-expressing cells of the ring gland in the background of schlank^{*G*0349} larval lethal mutants, it was possible to rescue the animals to adulthood. In contrast, expression of a schlank transgene in the fat body of schlank^{*G*0349} mutants by using ppl-Gal4:UAS-schlank causes partial rescue to the third larval instar stage (Figure 3). These data demonstrate that although schlank is ubiquitously expressed, its function is primarily required in vivo in the akh-expressing cells of the ring gland.

In search for the mechanism underlying the schlank-dependent transcriptional control of target genes regulating lipid homeostasis, the involvement of the putative homeodomain, which is located in the N-terminal part of the schlank protein has been tested. Firstly, an antibody specifically directed against the Hox domain of the schlank protein (sHox; Figures 4) has been generated and the third instar larvae has been analysed. In addition to the vesicular pattern observed with the C-terminal schlank antibody, a nuclear staining in the Akh-expressing ring gland cells has been determined in which schlank is functionally required, as determined by the tissue-specific rescue experiments. These data suggest that Hox domain-containing portions of schlank are present in the nucleic of ring gland cells. To investigate whether the Hox domain of schlank is generated by regulated processing events, biochemical experiments with extracts derived from larvae of different nutritional states has been performed.

The Drosophila schlank protein is composed of 400 amino acids and its predicted molecular weight is 46.2 kDa. Immunoprecipitation experiments using either HA-tagged versions of schlank in which the tag was located N-terminally, C-terminally or internally adjacent to the Hox domain, or by using the schlank sHox antibody (Figures 5A, 6) has been performed. In cellular extracts from well fed early third instar wild type larvae, a weak signal corresponding to full-length schlank protein and several smaller Hox domain-containing polypeptides, including 15 kDa fragments and a single 10 kDa band (Figure 5A left panel) has been observed. The analysis of larval nuclear extracts indicates that the 15 kDa and the 10 kDa polypeptides are present in the nucleus Figure 5A right panel, consistent with the immunohistochemical data obtained in the ring gland cells. Using the N-terminal HA-tagged version of schlank, it has been determined that the 15 kDa bands are derived from the most N-terminal part of the schlank protein, whereas the 10 kDa band seems to contain the homeodomain only, as determined by using the internal HA-tagged version of schlank in combination with the sHox domain antibody (Figure 6). Using cell extracts from starving larvae or from insulin receptor substrate mutants (chico mutants), which mimics a starved physiological state, it has been found that the protein cleavage pattern was changed; the amount of the 15 kDa homeodomain-containing polypeptides was strongly reduced and the 10 kDa band was absent. In summary, these data provide evidence for a regulated proteolytic cleavage of the schlank protein depending on the nutritional state. In the fed state, most of the schlank protein appears to be processed N-terminally to produce Hox domain-containing protein fragments with nuclear location. In contrast, upon starvation, cleavage of full-length schlank protein is reduced and the homeodomain-containing 10 kDa fragment is absent.

To further investigate the in vivo function of the schlank Hox domain, transgenic flies expressing a N-terminal fragment of schlank containing the Hox domain only without the C-terminal catalytic Lag1 motif that was shown to control (dihydro)ceramide synthesis in CerS family members (Pewzner-Jung et al., 2006, supra) has been generated (Figures 5B). When overexpressed in larvae, it has been found that the Hox domain-containing portion of schlank is able to downregulate the mRNA levels of the schlank target genes akh and lip3, and to upregulate the mRNA levels of FAS and ACC involved fatty acid biosynthesis, fully recapitulating the capacity of full-length schlank with respect to controlling transcription of target genes of the lipid metabolism (Figure 5C). These data strongly suggest a direct or indirect involvement of the schlank Hox domain in the regulation of target gene expression.

Homeodomain transcription factors have been extensively characterized as regulators of gene expression programs in development. Sequence analysis indicates that the Hox domain of schlank is most similar to homeodomains of the caudal family. However the schlank Hox domain, similar to the Hox domain of other Lass/CerS family members, contains a deletion of the first 15 amino acids of the 60 amino acid homeodomain sequence, as compared to the homeodomains of other known transcriptional regulators, Additionally, the putative third helix of the Hox domain of schlank does not contain a crucial phenylalanine required for DNA binding (the consensus motif WFQ is changed to WWR). Similar changes have also been found in the Hox domains of mammalian Lass/CerS proteins (Mesika et al., 2007, supra) suggesting that the Hox domain of Lass/CerS proteins are not capable to directly bind to DNA. Rather, this suggests that the schlank Hox domain may indirectly regulate target gene expression. To further investigate this, a yeast two hybrid screen to identify partners of the schlank Hox domain has been performed (Figure 6) and isolated the chromatin binding protein Df3 1 as a direct binding partner. Df3 1 was previously found to bind to histone H3 tails and to promote chromatin bridging (Guillebaut and Cotterill, 2007). These data further suggest that rather than binding to DNA directly, the Hox domain of schlank may interact with transcriptional regulators or chromatin regulatory proteins to modulate their function in the regulation of target gene expression.

Since the role of sphingolipids in cancer is established, intervening said lipid homeostasis system, thus, intervening the sphingolipid metabolism, by manipulating the expression of important molecules, in particular, enzymes involved in the sphingolipid metabolism, is of particular interest.

Consequently, interfering into said metabolism allows automatically manipulating the effects of high and low amounts of sphingolipids and, consequently, their effects on apoptosis and cell proliferation as described for example in Ogretmen, FEBS letters, 580 (2006), 5467 - 5476.

Thus, in another aspect, the present invention relates to a method for treating or preventing of lipid-based metabolic diseases, cancer (neuro)degeneration, autoimmunity, sepsis, cystic fibrosis, diabetes or modulating the immune system in an individual.

Polypeptides according to the present invention comprising the sequence of Seq. ID No. 1 or Seq. ID No. 2 or Seq. ID No.4 or 5 or 6 to 11 and not containing Seq. ID No. 3 or a functional fragment thereof of at least six amino acids, i.e. a cleaved fragment containing the homeodomain of the ceramide synthase but not containing the Lag domain, reflect a state with nutritional abundance and promotes the lipogenesis while upon starvation cleavage of the full length protein is reduced and the homeodomain containing fragment is substantially absent, hence, promoting the lipolysis pathway. For the first time, it has been demonstrated, that the homeodomain is cleaved off from ceramide synthase and, in addition, that a regulatory circuit linking sphingolipid and SREBP-dependent fatty acid and phosphorlipid biosynthesis, with insulin and akh-dependent body fat metabolism.

In addition, compounds altering the cleavage of the homeobox domain from the ceramide synthase protein would, as a consequence, influence the lipolysis and lipogenesis in an individual, i.e. the body fat metabolism as well as the fatty acid and phospholipid biosynthesis.

Hence, another embodiment of the present invention relates to a method for screening compounds altering the amount of cleaved fragment containing the homeodomain of an ceramide synthase enzyme containing said homeodomain. Said compounds are useful as pharmaceuticals for the treatment of lipid-based metabolic diseases, cancer (neuro)degeneration, autoimmunity, sepsis, cystic fibrosis, diabetes or for modulating the immune system in an individual; or as an anti aging compound. In particular, said compounds acting as an inhibitor of cleaving of the homeodomain from a ceramide synthase enzyme would be beneficial for treating diseases related to an overweight of the individual or of people suffering from adipositas or other related diseases.

Said inhibitors may be provided in form of a dietary supplement or as an ingredient of food.

In another embodiment, said inhibitor is a mutated polypeptide of the ceramide synthase enzyme containing said homeodomain and the Lag domain being mutated in so far, that said polypeptide does not contain a restriction site allowing cleaving off the homeodomain from the ceramide synthase enzyme. Said polypeptide contains a sequence of Seq. ID No. 1 or Seq. ID No. 2 and, in addition, of Seq. ID No. 3 whereby said polypeptide does not contain a restriction site allowing cleavage of the homeodomain containing fragment of Seq ID. No. 1 or 2 from the peptide of Seq ID No.3.

The polypeptides or nucleic acids may be provided in form of compositions. Said compositions are particularly adapted for systemic, but also topical administration to a subject. Particularly preferred, said composition is in form of a plaster, ointment or salve.

The present invention encompasses nucleic acid molecules encoding the polypeptides according to the present invention. Said nucleic acids may be in form of single or double stranded DNA or RNA molecules. Of course, molecules having altered structures, in particular being alternatively linked, e.g. by peptide bonds, are encompassed into the term nucleic acid molecules.

Said nucleic acids may be contained in a suitable vector. Said vector may allow expression of the nucleic acids and for the polypeptide encoded by said nucleic acids. Said vector maybe adapted for direct administration to a subject or may be a vector for expression of the polypeptide or the nucleic acid in known expression systems either prokaryotic or eukaryotic expression systems. The expression vector is a replicable or a non-replicable expression vector as known in the art.

The vectors of the present invention may be transformed or transferred into a host cell wherein the polypeptide is expressed.

Said host cells may be prokaryotic or eukaryotic host cells known in the art and transformation of said cells maybe effect by known methods.

The nucleic acid molecules either encoding a polypeptide according to the present invention or an isolated antisense RNA or DNA, silencer mRNA, RNAi, microRNA, non-coding RNA maybe obtained by chemical synthesis or by a known amplification technique.

In this connection the term "complementarity" or "complementary" between nucleic acids is the degree to which the bases in one nucleic acid strand can hydrogen bond or base pair, with the bases in a second nucleic acid strand. Sufficient complementary means that a sufficient number of the nucleotides in a first nucleic acid strand from base pairs with nucleotides in a second nucleic acid to generate a stable hybridization complex at about room temperature, i.e. at about 20°C to about 25°C.

Complementarity can sometimes be conveniently described by the percentage, i.e. proportion, of the nucleotides which can form base pairs between two nucleic acid strands or within a specific region or domain of the two strands. When expressed or measured by percentage of base pairs form, the degree of complementarily can range from at least about 50 % to full, namely, 100 % complementarily. Preferably, the complementarities of at least about 50 %, like 60 %, 70 %, 80 %, 90 %, 95 %.

The term "homology" as used here, is a degree of sequence identity between two nucleic acid strands or two polypeptide sequences. The degree of homology can also be described by the percentage of identical nucleotides or amino acids in two nucleotides or polypeptide sequences, respectively. In particular, the degree of homology between a target nucleic acid and a probe or oligonucleotide of the present invention can vary as long as selective hybridization is attained and range from at least about 50 % to about 100 % homology, like 60 %, 70 %, 80 %, 90 % or 95 % homology.

Using the antisense RNA or DNA, silencer mRNA, RNAi, microRNA or non-coding RNA complementary to selectively hybridize to nucleic acid molecules encoding the polypeptides of the present invention or to the DNA or mRNA encoding the ceramide synthase containing the homeodomain, thus, repressing the transcription and/or translation of the ceramide synthase and, hence, interfering the lipid hoemostasis as well as the insulin and akh-dependent body fat metabolism.

The isolated antisense RNA or DNA silencer mRNA, RNAi, microRNA or non-coding RNA complementary to selectively hybridize to the nucleic acid has a sufficient length and complementarily to hybridize to the target DNA, or mRNA, respectively.

Typically, these antisense molecules are oligonucleotides of a length of at least 10 nucleotides, like 12, 14 or 16 nucleotides, preferably at least 20 nucleotides or more in length, typically, the oligonucleotides and nucleic acids are of up to about 50 nucleotides length and can be chemically synthesized by available synthetic procedures for nucleic acids. Of course enzymatic methods are also available for DNA, RNA or oligonucleotide synthesis and the skilled person is well aware of suitable methods.

The polypeptides or nucleic acid molecules may be administered in form of compositions, in particular pharmaceutical compositions optionally containing suitable diluents, excipients or carriers. Typically, the compositions are administered systemically, for example topically or parenterally. The skilled person is well aware of suitable administration forms depending on the site of the pharmaceutical action.

### The following examples further illustrate the invention.

### General Procedures

### Fly Strains, Temperature Shift, and Rescue Experiments

Fly stocks were obtained from the Bloomington stock center. The following *schlank* alleles were used: P{lacW}I(1)G0061^{G0061} (*schlank^{G0061}*, Bloomington stock 11665) and P{lacW}I(1)G0349^{G0349} (*schlank^{G0349}*). The allele P{lacW}I(1)G0370^{G0370} (*schlank*^{*G06370*)} shows the same phenotype as P{lacW}I(1)G0349^{G0349}, however, it was not further used in this study. *schlank^{G0370}* and *schlank^{G0349}* were isolated by screening the Göttingen X-chromosome collection of P lines. In all three *schlank* alleles, the P elements are integrated in the first exon of the gene, as shown in Figure 3, and quantitative RT-PCR experiments show that *schlank* mRNA levels are reduced in the *schlank* alleles (hemizygous *schlank^{G00061}* larvae show a moderate reduction and hemizygous *schlank^{G0341}* larvae a strong reduction of *schlank* mRNA levels, as compared to wild type (Figure 4C). For phenotypic analysis all *schlank* alleles were balanced over *FM7c,* P{*Kr*-Gal4}, P{UAS-*GFP*} and mutant larvae were scored based on loss of fluorescence detection. *schlank* mutant clones in the germline were generated by recombination of P{lacW}I(1)G0349^{G0349} onto the FRT101 chromosome. Females of the genotype *schlank^{G0349}*, *FRT101*/*FM7c* were crossed with *w ovo^{D1} v P{FRT(w^{hs})}101*/*Y; P{hsFLP}38* males. Larvae were heat shocked after 48h and 72h for 2h at 37°C. Females of the genotype *w schlank^{G0349}, FRT101*/ *w ovo^{D1} v P{FRT(w^{hs})}101* were selected and crossed to *FM7c*, P{*Kr*-Gal4}, P{UAS-*GFP*} males. No eggs were obtained from these crossings, indicating that maternal clones homozygous for *schlank^{G0349}* die during oogenesis.

The growth and the specific metabolic defects observed in the *schlank* P-alleles could be phenocopied when *schlank* mRNA levels were downregulated in larvae and in tissue culture cells using RNAi-mediated knock down of the gene (Figures 1, 3, 7). To this end, transgenic flies expressing a *schlank* RNAi construct (w1118; pMF3 DLAG1 RNAi #33896 or w1118; pMF3 DLAG1 RNAi #41114VDRC stock center) were used in combination with a *daughterless (da)*-GAL4 driver line mediating ubiquitous expression. The *schlank* RNAi line is specific for the knock down of *schlank* and no "off target" effects are observed (VDRC at http://www.vdrc.atæ).

For rescue experiments, flies of the genotype, *schlank^{G0349}*/ *FM7c,* P{*Kr*-Gal4}, P{UAS-*GFP*}; +; UAS-*schlank-HA* were crossed to the *akh* promoter-*GAL4* transgene (akh-Gal4) or to the *pumpless (ppl)*-promoter-*GAL4* transgene to express a full length *schlank* cDNA in a *schlank* mutant background. Rescues were verified by genomic PCR using schlank F1 and Plac1 primers (see below) to check for P element insertion. Mobilization of the P elements P{lacW}I(1)G0370^{G0370}, P{lacW}I(1)G0349^{G0349}, and P{lacW}I(1)G0163^{G0163}, to induce precise excision could completely rescue from lethality. *hs*-Gal4 was combined with UAS-*schlank-HA* for ubiquitous expression from a heat shock promotor. For overexpression in Akh producing cells and fat body *akh*-Gal4 and *ppl*-Gal4 were used. As controls we used w¹¹¹⁸ flies, which were also hosts for generating transgenes. To stain the AKH-producing cells, the akh-GAL4 was crossed to UAS-*mCD8-GFP.* Analysis of oenocytes in *schlank^{G0061}* mutants was performed by crossing *schlank^{G0061}*/*FM7c*, P{*Kr*-Gal4}, P{UAS-*GFP*} flies with flies carrying Gal4 *109(2)80*, UAS-*mCD8-GFP*/ Gal4 *109(2)80*, *UAS-mCD8-GFP* on the second chromosome. For staining experiments we scored for *sch*/*ank^{G0061}*/ Y; Gal4 *109(2)80*, UAS-*mCD8-GFPI* + larvae. For staining of lipid droplets we used *w*[*]; +; P{w_mC UAS-Lsd-*2-EGFP*}*l* TM3 Sb[1]e[1] (*Lsd-2-EGFP*) and *w*[*]; P{w_mC UAS-*Bmm-EGFP}l* CyO (*Bmm-EGFP*). The following insulin signalling mutants and UAS constructs were used: *Steppke (step)* P{lacW}I(2)k08110^{k08110}, chico¹ (gift I. Hahn), *FOXO²¹* and *FOXO^{W24}*, UAS-*FOXO* and *UAS-FOXO^{™}* lines.

### Cloning and Transgene Production

*A schlank* fragment corresponding to the full-length *schlank* cDNA (Genbank accession number LD18904) was cloned into the pP{UAST} transformation vector via EcoRI/XhoI to generate the pP{UAS-*schlank*} (UAS-*schlank*) transgene. *schlank-CT-HA*, *schlank-NT-HA, schlank-HA-Hox, schlank-Myc*, and *Flag-schlank* was generated by PCR reaction from LD18904 containing the entire *schlank* cDNA using specific *schlank* primer pairs. The tag sequence is printed in bold letters. *schlank-CT-HA, schlank-NT-HA, and schlank-Myc* were inserted into pP{UAST} using EcoRI/XbaI to produce pP{UAS-*schlank-CT-HA*} (UAS-*schlank-CT-HA*), *pP*{UAS-*schlank-Myc*} (UAS-*schlank-Myc*) and pP{UAS-*schlank-NT-HA*} (UAS-*schlank-NT-HA*) transgenic flies. To generate pP{UAS*-schlank-HA-Hox*} having the HA-tag in front of the Hox domain, pP{UAS-*schlank*} was digested with EcoRV, which cuts twice within the *schlank* cDNA at position 194 and 543 (relative to the first ATG) just 27 bp before the Hox motif and 66 bp before the Lag motif leading to the release of a 349 bp fragment. The *schlank-HA-Hox* amplification product was digested with EcoRV too. This fragment was then used to replace the 349 bp fragment within pP{UAS-*schlank*} leading to a pP{UAS-*schlank-HA-Hox*}, which has a HA-tag 21 bp (7 amino acids) in front of the Hox domain. Cloning of pP{UAS-*schlank-HA-Hox*} (UAS-*schlank-HA-Hox*} was verified by sequencing. The pP{UAS-*schlank*}, pP{UAS-*schlank-HA-Hox*}, the pP {UAS-*schlank-CT-HA*}, pP {UAS-*schlank-NT-HA*}, and pP{UAS-*schlank-Myc*} constructs were integrated into w⁻ fly genomes by P element-mediated transformation. *schlank-Myc* or was inserted into pMT/V5 using EcoRI/XbaI to produce pMT/V5-*schlank-Myc* (pMTV-*schlank-Myc*). pMT/V5-*schlank-Myc* carries a *schlank* cDNA under the control of a copper-inducible promoter (pMT/V5 plasmid from; Invitrogen, Carlsbad, CA). Primers were as follows:

| | |
|---|---|
| *schlank* start: | CGGGATCCCAACATGGACATATTGAATGAGTTCAG (Seq. ID. No. 14); |
| *schlank-CT-HA*: | GCTCTAGATTAAGCGTAATCTGGAACATCGTATGGGTACTCCTCT |
| | CTCCGTTCCGTTGTCGCAA (Seq. ID. No. 15); |
| *schlank-NT-HA*: | GGAATTCAACATGGACTACCCATACGATGTTCCAGATTACGCTATA |
| | TTGAATGAGTTCAGCAATGTA(Seq. ID. No. 16); |
| *schlank-HA-Hox*: | TGGATATCGTACCCATACGATGTTCCAGATTACGCTCCCGTAG |
| | GCAAATCACTTGGCATACG (Seq. ID. No. 17); |
| *schlank-Myc*: | CCGCTCGAGCGGTTACAGATCCTCTTCAGAGATGAGTTTCTGCTC |
| | CTCCTCTCTCCGTTCCGTTGTCGCAA (Seq. ID. No. 18); |
| *schlank* stop: | CCGCTCGAGCTACTCCTCTCTCCGTTCCGT (Seq. ID. No. 19): |

### Size Measurement and Statistics

Embryos were collected for 1 hours at 25°C on apple juice agar plates containing yeast paste and were allowed to develop. Length of larvae was determined 24, 48, and 72 hours after egg laying. Values are presented as mean ± SEM. Data were checked for normal distribution, and P-values were calculated using the two-tailed Student's t-Test for two samples with unequal variances following normal distribution. For datasets with too few samples for a t-Test, Kruskal-Walis-Test was used to verify differences in mean values. P-values <0.05 were considered to be statistically significant (P<0.001 designated by triple asterisks).

### Triacylglycerol (TAG) Assay.

Relative TAG and total protein content was quantified as described previously (Grönke et al., Cell Metab. 1, 323-330, 2003) with the following modifications: Using morphological criteria 10 wandering third instar stage larvae were homogenized in TAG lysis buffer. Following centrifugation at 13200 rpm for 5 min in a tabletop centrifuge at 4° C, the supernatant was transferred to a fresh tube, avoiding debris. To measure the TAG content, 50 µl of the resulting supernatant was combined with 1 ml of TAG Reagent (Thermo Electron Corp.), incubated for 15 min at 37 °C and OD₅₄₀ₙₘ was determined. To measure protein levels, 50 µl of the resulting supernatant was combined with 950 µl Pierce BCA Reagent Mix, incubated at 37 °C and OD₅₉₅ₙₘ was determined and compared with a standardization curve. OD₅₄₀ₙₘ values per total protein content of mutant larvae were normalized to mean OD₅₄₀ₙₘ values per total protein content of wildtype larvae. Mean values of at least 2 independent experimental setups were calculated and those experiments were repeated twice. Values show the experimental mean with the corresponding SEM.

### Temperature Shift Experiments

For *schlank* overexpression 0 to 4 h embryo collections of the genotype UAS-*schlank-HA; hs*-Gal4 were incubated at 25 °C for 72 h. Larvae were heat shocked for 1 hour at 37 °C and incubated for another 3 hours at 25 °C before RNA isolation. *schlank* transcripts were induced 15 to 20 fold (data not shown).

***schlank* dsRNA:** To generate *schlank* dsRNA, a fragment of the *schlank* cDNA was generated by PCR reaction from LD18904 containing the entire *schlank* cDNA using specific *schlank* primer pairs containing T7 sites and cloned into the Topo pCRII vector (Invitrogen Corp., Carlsbad, CA). The following primers were used:

| | |
|---|---|
| *schlank* RNAiF1: | CTTTTAATACGACTCACTATAGGGAGACGTTTCTGGATA |
| | TCGCCCGTAGGCAAATC (Seq. ID. No. 20) |
| *schlank* RNAiR1: | GTTTTAATACGACTCACTATAGGGAGACAGACCCTTCTGCAGAG |
| | AGTCGACAACAA (Seq. ID. No. 21) |

dsRNA was synthesized using RiboMax Large Scale RNA Production System - T7 - kit (Promega) according to the supplier's instructions. After synthesis, annealing and RNAse (specific for single stranded RNA)/DNAse treatment, dsRNA was purified by chloroform/isoamylalcohol extraction and precipitated using standard sodiumacetate/isopropanol precipitation. The dsRNA was analyzed on a 1% TAE-agarose gel to ensure that at least 80-90% of the RNA is double stranded. Micromolar concentration was determined with Oligonucleotide Properties Calculator (Northwestern University).

### Tissue Culture and Transfection

*Drosophila* Schneider cells (SL2) were propagated in Schneider's medium (PAN) supplemented with 10% heat inactivated fetal bovine serum and 1% penicillin/streptomycin (Sigma, St Louis, USA). For RNAi-treatment 2x10⁶ cells were placed in serum-free medium containing *schlank* dsRNA and incubated for 30 min. Afterwards the same volume of complemented medium was added and cells were grown for additional 3 days at 25 °C before RNA isolation for qRT-PCR. Final dsRNA concentration was 75 nM. For lipid analysis 2x10⁶ SL2 cells were treated with dsRNA (75 nM) for 4-5 days. Eventually, the medium is replaced by fresh medium containing dsRNA (75 nM) and further 5-6 days incubation at RT to allow a complete lipid turnover. For transfection experiments 2x10⁶ cells were plated in a 35- mm diameter multi well plate or in 100 mm dishes to attach overnight, transfected with 1 µg *schlank-Myc* or *schlank-Myc* H215D plasmid or with 4 µg *Flag-schlank* using Effectene reagent according to the manufacturer's instructions (Qiagen). Expression was induced for both with mock and with *schlank* plasmid transfected cells with 0.7 µM CuSO₄ for 72 h.

### Antibody Generation

Polyclonal antisera were generated in rabbit (Davids Biotechnology, Regensburg, Germany) and in guinea pig (BioGenes, Berlin, Germany) against schlank-specific oligopeptides conjugated to LPH and to KLH, respectively. The peptides used were RSSRPKKAANVPI (Seq. ID No. 22) representing amino acids 75-87 and SRAGARVATTERREE Seq. ID No. 23) representing amino acids 297-310 of schlank. IgGs were purified from immune serum by affinity purification. The antibodies directed against the amino acids 75-87 and 297-310 were named schlank Hox (sHox) and schlank CT (sCT), respectively.

### Immunohistochemistry

For antibody staining, larvae were dissected in phosphate buffered saline (PBS) and fixed overnight in 4% formaldehyde/PBS + 0.3% Triton X-100 (PBT). Primary antibodies were added for 4 h in PBT containing 2% goat serum followed by the incubation with secondary antibodies for 1.5 h. The following stains and primary antibodies were used: anti-schlank CT (sCT; guinea pig, 1:40), anti-sHox (sHox; rabbit, 1:40), monoclonal anti-c-Myc 9E10 (1: 100, Santa Cruz), anti- Spectrin (mouse, 1:50; Developmental Studies, Hybridoma Bank), anti-GFP (mouse, 1:100, Santa Cruz), anti-DE-cadherin (rat, 1:50; Santa Cruz Biotechnology, Santa Cruz, CA), anti-Membrin (rabbit, 1:250; Stressgen), and anti-Rab11 (rabbit, 1:250; gift of D. F. Ready). The following secondary antibodies were used: Alexa Fluor 488 (1:200; Molecular Probes), Alexa Fluor 633 (1:100; Molecular Probes), Cy3 (1:200; Dianova), Cy2 (1:100; Dianova), Cy5 (1:100; Dianova).

SL2 cells were fixed in 4% paraformaldehyde for 10 min at room temperature (RT), washed with PBS containing 1% BSA (PBSB), and permeabilised with PBS supplemented with 0,2% Triton X-100 for 5 min. Following washing steps with PBS and PBSB, SL2 cells were stained with the corresponding primary antibodies in PBSB for 30 min at RT. After extensive washing with PBSB at RT, the samples were processed for indirect immunofluorescence by incubation with the corresponding secondary fluorescent antibody for 30 min at RT. The samples were mounted in Vectashield mounting medium. Fluorescent images were recorded using a Leica TSP2 confocal microscope (Leica, Wetzlar, Germany), and images of multilabeled samples were acquired sequentially on separate channels. All images were processed with Adobe Photoshop software and assembled using Adobe Illustrator software.

### Immunoprecipitation and Immunoblotting

We used larval extracts of 0 to 6 h embryo collections of the genotype UAS-schlank-*CT-HA; hs*-Gal4, UAS-*schlank-NT-HA; hs*-Gal4, and UAS-*schlank-HA-Hox; hs*-Gal4, which were incubated at 25 °C for 68-72 h. Larvae were heat shocked for 1 h at 37 °C and incubated for another 3 h at 25 °C before protein extract preparation.

To compare the schlank protein pattern of starved and fed larvae, 0 to 6 h embryo collections were used, which were incubated at 25 °C for 60 h followed by a 12 h incubation on either apple juice agar plates well supplied with yeast or placed on filter paper on a Petri dish soaked with phosphate-buffered saline (PBS). *Drosophila* larvae were homogenized in RIPA buffer (150 mM NaCl, 1% IGEPAL CA-630, 0,5% Sodium Deoxycholate, 0,1% SDS, 50 mM Tris (pH 8,0), supplemented with protease inhibitors (Complete^{™}; Roche, Indianapolis, USA), and centrifuged at 10,000 rpm for 10 min in a tabletop centrifuge at 4 °C. The supernatant was further used for determination of protein content, immunoblotting and immunoprecipitation. Whole cell larval *Drosophila* lysates (50 µg of protein) were separated by 15 % SDS-PAGE and transferred to a PVDF-membrane (Immobilon P transfer-membrane; Millipore). The immunoblot membrane was blocked with non-fat dry milk in TBS plus 0.05% Tween (TBST) and then incubated with either anti sHox (1: 500), anti-Myc (1:400) or anti-HA (1:500). After washing, bound antibody was visualized with peroxidase-conjugated donkey anti-rabbit, donkey anti-mouse or donkey anti-rat IgG (all 1:15000) using the ECL system (Amersham Pharmacia). Co-immunoprecipitation was performed using the immunoprecipitation starter pack (Amersham Biosciences, Piscataway, NJ) according to the manufacturer's instruction. To avoid nonspecific binding of proteins to A/G-Sepharose, preclearing of the cell-lysates was performed in the presence of protein A/G-Sepharose according to the manufacturer's manual. The cleared supernatant was incubated for 12 h with anti-sHox or anti-HA antibody in a final volume of 500 µl. 40µl protein A/G- Sepharose was added to each mixture and incubated for 4 hours. The beads were washed 3 times with excess RIPA-buffer and 2 times with excess PBS, resuspended in SDS-Page sample buffer, and boiled for 3 min. Samples were run on a 15 % SDS-gel, and transferred to a PVDF-membrane (Immobilon P transfer-membrane; Millipore). The immunoblot membrane was treated as described above.

### RNA Isolation and Realtime PCR

*Drosophila* larvae or SL2 cells (for larvae more than 200 animals) were transferred to lysis buffer after washing. Homogenisation was performed in Ultra-Turrax T25basic homogeniser at full speed two times for 30 seconds followed by total RNA isolation with a NucleoSpin RNA II kit (Macherey and Nagel). Using the QuantiTect Reverse Transcription kit (Qiagen) cDNA was prepared after Dnasel treatment according the manufacturer's instructions. *Realtime* PCR was performed in a reaction mix containing cDNA template, primer pairs and iQ SYBR Green Supermix (BIO-RAD) in a final volume of 25 µl. For each template reactions were done in parallel and repeated at least twice with independently isolated RNA samples from different experimental set ups. Expression of *actin* 5C (*Act5C*, *act*) and *Ribosomal protein L32 (RpL32, rp49*) was used as reference for normalization. The experiments were performed in a iQ5 Real-Time PCR Detection System from BIO-RAD. Analysis of the *realtime* PCR was done using BIO-RAD iQ5 Optical System software (version 1.1.1442.OCR), according the manufacturer's instructions, and Microsoft Excel. The following primers were used for *realtime* PCR analysis:

| | |
|---|---|
| *Act5C* F1: | GTGCACCGCAAGTGCTTCTAA (Seq. ID. No. 24) |
| *Act5C* R1: | TGCTGCACTCCAAACTTCCAC (Seq. ID. No. 25) |
| *RpL32* F1: | GCTAAGCTGTCGCACAAATG (Seq. ID. No. 26) |
| *RpL32* R1: | GTTCGATCCGTAACCGATGT (Seq. ID. No. 27) |
| *schlank* F1: | GAATGCACGTCTGACCAATG (Seq. ID. No. 28) |
| *schlank* R1: | TTGTCGCAACTCTTGCTCCTG (Seq. ID. No. 29) |
| *Lip3* F1: | TGAGTACGGCAGCTACTTCCCT (Seq. ID. No. 30) |
| *Lip3* R1: | TCAACTTGCGGACATCGCT (Seq. ID. No. 31) |
| *Bmm* F1: | ACGCACAGCAGCGACATGTAT (Seq. ID. No. 32) |
| *Bmm* R1: | CTTTTCGCTTTGCTACGAGCC (Seq. ID. No. 33) |
| *Akh* F1: | GGTCCTGGAACCTTTTTCGAG (Seq. ID. No. 34) |
| *Akh* R1: | TTGCACGAAGCGGAAGATC (Seq. ID. No. 35) |
| dILP2 *F1*: | CTGAGTATGGTGTGCGAGGA (Seq. ID. No. 36) |
| dILP2 *F1*: | GCGGTTCCGATATCGAGTTA (Seq. ID. No. 37) |
| *InR* F1: | AACAGTGGCGGATTCGGTT (Seq. ID. No. 38) |
| *InR* R1: | TACTCGGAGCATTGGAGGCAT (Seq. ID. No. 39) |
| *DSREBP* F1: | CGCAGTTTGTCGCCTGATG |
| *DSREBP* F1: | CAGACTCCTGTCCAAGAGCTGTT |
| *FAS* F1: | CCCCAGGAGGTGAACTCTATCA |
| *FAS* R1: | GACTTGACCGATCCGATCAAC |
| *FCS* F1: | CATCGCCCAGCGTAGCATA |
| *FCS* R1: | CGAGCCGAATGGATGATAGC |
| *ACS* F1: | AAATCCCATGGACCGATGAC |
| *ACS* R1: | TGTAGAGCATGAACAATGGATCCT |
| *ACC* F1: | GTGCAACTGTTGGCAGATCAGT |
| *ACC* F1: | TTTCTGATGACGACGCTGGAT |

*DSREBP, FAS,* ACC, *ACS and FCS* primers were described previously (Seegmiller at al., 2002,Dev. Cell, 2, 229-238).

### (Dihydro)ceramide Synthase Assay

For the assay we used larvae of the strong hypomorphic *schlank^{G0349}* line and wildtype (WT) larvae 48 h after eclosure (AEC), and larvae (48 h AEC) expressing UAS-*schlank-HA* ubiquitously after the application of a 1 h heat shock followed by an 3 incubation at 25 °C. Expression of UAS-*schlank-HA* was tested by immunoblot showing a HA signal already 1 after the heat shock (data not shown). The larvae were lysed in buffer A (50 mM Hepes/NaOH, pH 7.5, 0.5 mM DTT, 2x protease inhibitor (Complete^{™}; Roche, Indianapolis, USA) by sonication, and centrifuged at 10,000 rpm for 10 min in a tabletop centrifuge at 4 °C. The supernatant was transferred to a new tube. Protein content was measured with 4 µl and 2 µl of the supernatant combined with 46 µl and 48 µl buffer A in 950 µl Pierce Assay reagent (BCA Protein Assay Kit, Pierce). Following incubation for 30 min at 37 °C, OD₂₆₀ was determined and compared with a standardization curve. 40 µg of protein were mixed with 5 µM dihydrosphingosine (Sigma, Germany), 0.2 µCi of [4,5 -³H] D-erythro-dihydrosphingosine (82 Ci/mol) and 25 mM fatty arachidoyl-CoA in buffer B (50 mM Hepes/NaOH, pH 7.5, 0.5 µM DTT, 1 mM MgCl₂, 0.1 % digitonin) in a volume of 100 µl. After incubation for 20 min at 37 °C, the reaction was stopped with 100 µl of chloroform/methanol (1:1, v/v). The dried lipids were resuspended in 40 µl of chloroform/methanol (1:1, v/v) and separated on Silica Gel60 HPTLC plates (Merck). The separated lipid bands were visualized with a phosphoimager (FUJIX Bio Imaging Analyser 1000, Fuji Photo Film, Tokyo, Japan) and quantified by TINA 2.08 software (Raytest, Straubenhardt, Germany).

### Lipid Analysis in Larvae

Larvae were homogenized, lyophilized and weighed. Lipids were extracted in chloroform/methanol/water (2:5:1 v/v/v). Unpolar lipids (ceramide, sterol, fatty acid, triacylglycerol) were separated by TLC with chloroform/methanol/glacial acetic acid (190:9:1, v/v/v). For quantitative analytical thin layer chromatography determination, increasing amounts of standard lipids (ceramide (C18:1/C18:1), cholesterol, trioleoylglycerol (all Sigma), and stearic acid (Fluka)) were applied. Plates were air dried, sprayed with 8% (w/v) H₃PO₄ containing 10% (w/v) copper (II) sulfate pentahydrate, and charred for 10 min at 140 °C for sterol and for 180 °C for the other lipids, and lipids were quantified by photo densitometry (Shimadzu, Kyoto, Japan) at 595 nm.

Also, the individual ceramide fractions were analyzed by ESI-mass spectrometry (ESI-Q-Tof 2 mass spectrometer equipped with a nanospray source, MicroMass, Manchester, UK). Aliquots of the lipid extract were separated as described above. The different ceramides were scraped from the TLC plate and reextracted in chloroform/methanol 1:1 (v/v). The solutions were injected to the mass spectrometer by glass capillaries using a capillary voltage in negative ion mode of 1200 V and in positive ion mode of 1000 V and a cone voltage of 50 V at 80°C. Instrument calibration was done with a mixture of sodium iodide and cesium iodide in propanol/water 1:1 (v/v). For MS/MS experiments argon was used as collision gas, and fragmentation was observed at energy values from 20-50 eV.

### Lipid Analysis in SL2 Cells

After treatment with dsRNA for up to 11 days the medium was replaced by fresh serum-free medium containing dsRNA (75 nM) and [¹⁴C]serine (1 µCi/ml). The biosynthetic labelling and the extraction of the lipids were performed as described in the art. For the separation of ceramides and dihydroceramides, TLC plates impregnated with borate were used and developed in chloroform/methanol 90:10 (v/v). Radioactive spots were evaluated and quantified using the bioimaging analyzer Fuji-Bas 100 and the TINA 2.08 software (Raytest, Straubenhardt, Germany).

Lipid extracts of SL2 cells were analyzed by means of LC/APCI-MS, the LC system was a 2695 Alliance separation module (Waters, Eschborn, Germany). In addition, The LC system was coupled to a Q-TOF 2 mass spectrometer. The mass spectra shown in Figure 7 were performed in the full scan mode (m/z: 400-1500). For detailed structural information LC/APCI-MS/MS has been carried out. The collision energy was 50V.

### Yeast Two-Hybrid Analysis and Plasmid Construction

For the construction of the bait vector, a DNA fragment (base pairs 195 -432; amino acids 65-144) containing the Hox domain (base pairs 219-396; amino acids 73-132) of *schlank* was cloned into the CytoTrap bait vector pSOS (Stratagene), which is further referred to as *schlank-Hox.* The *schlank-Hox* PCR-product was generated using the LD 18904 clone as template together with the following primers:
*schlank* Hox F1: CGGGATCCTGATATCGCCCGTAGGCAAATCA (Seq. ID. No. 40) *schlank* Hox R1:ATAAGAATGCGGCCGCGATGCAACGCCACGTGTTCTC (Seq. ID. No. 41)

Subsequently, the *schlank* PCR-amplification product was cloned in frame with hSOS into the bait vector via the 5'-BamHI and 3'-Notl restriction sites. The schlank Hox/hSOS fusion protein was tested for auto activation using 100 µg of an embryonic *Drosophila melanogaster* cDNA library (Stratagene) together with 100 µg of the bait construct for transformation into the yeast S. *cerevisiae* temperature-sensitive mutant strain cdc25H.

The CytoTrap Screen was performed according the CytoTrap Manual (Stratagene) instructions. A mutation within the cdc25-gene allows growth at 25°C and prevents growth at 37°C. The CytoTrap system is based on the ability of the hSos, to complement the cdc25 defect and to activate the yeast Ras signaling pathway. The mutation can be complemented by recruiting the *hSOS* gene product to the plasma membrane. Localization of hSos to the plasma membrane occurs through the interaction with the target proteins, which are localized to the plasma membrane by a fused myristylation sequence. After first screening procedures and incubation at 37°C for 5 days 31 target clones were isolated. All, but one was confirmed in a rescreen. The target clones were further tested for lack of auto activation. After DNA isolation and restriction enzyme analysis 6 independent clones were analysed by sequencing among them a clone coding for the *Df31* chromatin decondensation factor. The interaction of schlank with DF31 was further confirmed by using the *in vitro* glutathione S-transferase (GST) pull down assay. GST alone and GST-fusion to schlank-Hox were expressed, purified and tested for interaction with *in vitro* translated Df31.

## Claims

1. An isolated polypeptide comprising the sequence of Seq. ID No. 1:
RRX₁QX₂P (Seq. ID No.1)
wherein X₁ and X₂ are any amino acid,
for the prevention or treatment of lipid-based metabolic diseases, cancer, (neuro)degeneration, autoimmunity, sepsis, cystic fibrosis, diabetes or modulating the immune system in an individual; or as an anti aging compound.

2. The polypeptide according to claim 1 comprising the sequence of Seq. ID No. 2:
WX₃RX₄RRX₁QX₂P (Seq ID No. 2)
wherein X₁, X₂, X₃ and X₄ are any amino acid, or a functional fragment thereof of at least 6 amino acids.

3. An isolated polypeptide comprising Seq. ID No. 1 or 2 and not containing Seq. ID No. 3, or a functional fragment thereof of at least 6 amino acids having a ceramide synthase activity.

4. The polypeptide according to any one of the preceding claims having the sequence of Seq. ID Nos. 6 to 11.

5. A polypeptide containing a sequence of Seq ID No. 1 or Seq ID No. 2, and, in addition, of Seq ID No. 3 **characterized in that** said sequence does not contain a restriction site allowing cleavage of said polypeptide into a first fragment containing a homeodomain of Seq. ID No. 1 and a second fragment containing the Lag fragment of Seq. ID No. 3.

6. A nucleic acid encoding a polypeptide as defined in any one of claims 1 to 5.

7. A vector comprising a nucleic acid of claim 6.

8. A host cell comprising the vector of claim 7.

9. An isolated antisense RNA or DNA, a silencer mRNA, RNAi, microRNA, or non-coding RNA complementary to selectively hybridize to a nucleic acid according to claim 6.

10. A method for screening compounds for preventing or treating lipid-based metabolic diseases, cancer, (neuro)degeneration, autoimmunity, sepsis, cystic fibrosis, diabetes or modulating the immune system in an individual; or as an anti aging compound comprising the steps of
a) providing a cell line expressing a ceramide synthase containing a homeodomain comprising a sequence of any one of Seq ID Nos. 1 ,2, 4, 5 to...;
b) incubating the cells of a) in the presence and absence of compounds to be tested;
c) determining altered expression of the ceramide synthase or fragments thereof, altered posttranslational modification of the ceramide synthase, or the presence of cleaved homeodomain in cells incubated with the compound to be tested compared to the cells incubated in the absence of the compound to be tested.

11. A method for screening compounds according to claim 10 comprising the step of determining the presence of a peptide as defined in any one of claims 1 to 5 in the nucleus of said cells.

12. The method according to claim 11 wherein said compounds alter the proteolytic cleavage of the N-terminal homeodomain from the C-terminal end of a ceramide synthase.

13. Method for identifying compounds useful as pharmaceuticals for the treatment of lipid-based metabolic diseases, cancer, neurodegeneration, cystic fibrosis, diabetes, autoimmunity, sepsis and as anti-agent compounds as well as modulating the immune system of an individual, comprising the step of determining the ability of altering the amount of the cleaved homeodomain of a ceramide synthase enzyme containing said homeodomain.

14. Dietary supplement or food containing a compound according to any one of claims 3 to 5, or obtained by a method of claims 11 to 13.

15. Composition containing a compound according to any one of claims 1 to 5 adapted for systemic administration to a subject, preferably in form of a plaster, ointment, tablet, pill, injection, infusion or salve.
